(19) Europäisches Patentamt
European Patent Office
Office européen des brevets

(11) EP 4 768 470 A1

(12) **EUROPEAN PATENT APPLICATION**

(43) Date of publication:
**01.07.2026 Bulletin 2026/27**

(21) Application number: **25169843.7**

(22) Date of filing: **10.04.2025**

(51) International Patent Classification (IPC):
***C07C 301/00*** *(2006.01)* ***C07D 307/20*** *(2006.01)*
***A01N 41/02*** *(2006.01)* ***A01N 43/08*** *(2006.01)*

(52) Cooperative Patent Classification (CPC):
**C07C 301/00; A01N 41/02; A01N 43/08; A01P 3/00; A01P 7/02; A01P 7/04; C07D 307/20;**
C07B 2200/07

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **30.12.2024 CN 202411977822**

(71) Applicant: **Chengdu Hanova BioSciences Co., Ltd**
**Chengdu, Sichuan (CN)**

(72) Inventors:
- **CHEN, Tingzhuo**
  **Chengdu (CN)**
- **LIU, Jiayu**
  **Chengdu (CN)**
- **WANG, Zhouyu**
  **Chengdu (CN)**
- **SHI, Quan**
  **Chengdu (CN)**
- **WANG, Chao**
  **Chengdu (CN)**
- **YANG, Yang**
  **Chengdu (CN)**
- **LI, Shiqi**
  **Chengdu (CN)**
- **XU, Lei**
  **Chengdu (CN)**
- **HE, Qiming**
  **Chengdu (CN)**

(74) Representative: **Murgitroyd & Company**
**165-169 Scotland Street**
**Glasgow G5 8PL (GB)**

(54) **CHIRAL ISOMERIC SULFITE COMPOUND AND APPLICATION THEREOF**

(57) Chiral isomeric sulfite compounds, and preparative separation and application of chiral structures thereof are disclosed,.which belong to the technical field of agriculture. The present disclosure has found that the chiral isomeric sulfite compounds having the structure of general formula (A) have excellent inhibitory activities against pest eggs, especially have strong inhibitory activities against eggs of thrips, mites, Lepidoptera moths, Aleyrodidae and Harmonia axyridis, and meanwhile have germicidal activity, and their absolute configuration are obtained through preparative separation. The compounds with such structures can be used for killing pest eggs and germs, and have high research value of agrochemical, having broad application prospects in the field of agricultural pharmacy.

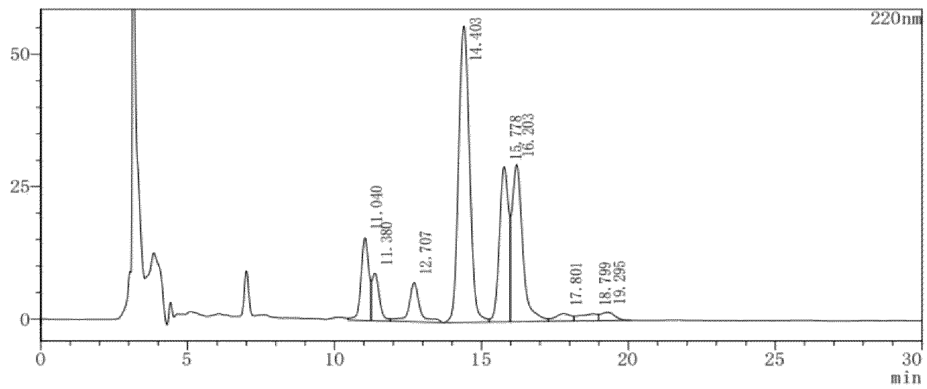


Figure 1



Processed by Luminess, 75001 PARIS (FR)

## Description

### TECHNICAL FIELD

The present disclosure relates to the field of agricultural technology, in particular to an insecticidal compound having a chiral isomeric sulfite, and preparative separation and application of a chiral structure thereof.

### BACKGROUND

The insecticide market is vast and huge, and has a market capacity of approximately tens of billions. Typical agricultural insecticides mainly include Orthoptera such as locusts and mole crickets; Hemiptera pentatomids; Homoptera including aphids, leafhoppers, planthoppers, etc; Thysanoptera Thrips; Various beetles of Coleoptera; Lepidoptera moths and butterflies; Hymenoptera bees and ants; Diptera mosquitoes, flies, and Tabanidae; various species of red spiders of Acarina.

In the existing prevention and control concept, the vast majority of insecticide products focus on the contact killing and stomach poisoning of imagos, nymphs, or larvae. By significantly reducing the number of insect populations on crops, the pest population base can be controlled, thereby protecting crops from excessive damage and reducing economic losses after the insect infestations occur. This method can usually effectively control the population base of pests in the environment, but it often cannot achieve very good effects in the prevention and control practices of some short-generation-cycle pest species such as various species of red spiders, thrips, etc.

Taking red spiders as an example, there are numerous types of registered acaricides, including dozens of plant sources such as Cyetpyrafen, Avermectin, Azocyclotin, Fenbutatin-oxide, Spirodiclofen, Etoxazole, root and stem extract of Veratrum nigrum L., and matrine, etc., inorganic mineral or chemical acaricides. However, most of these acaricides are targeted at adult mites or young nymph mites, and only some of them have good egg killing activity. In the field, the breeding speed of pest mites is extremely fast, with a single passage completed almost every other week. Therefore, the phenomenon of overlapping of generations is very serious. Therefore, when acaricides without egg killing effects are sprayed solely, although the adult mites and nymph mites are killed, new young mites will soon hatch from the eggs and rapidly expand the number of population. Although it is possible to clear away the pest mites that hatch later through secondary application, there may be some problems in practical application. For example, if pesticides are applied too frequently, the resistance of pest mites to insecticides will increase rapidly At the same time, the cost of pesticide application for farmers will also increase exponentially. In the field, in order to save labor costs, the traditional pesticide application methods tend to mixed application of multiple pesticides, such as barrel mixing of acaricides, insecticides, bactericides, regulators, foliar nutrition, etc. In the actual prevention and control application of pest mites in the field, farmers will choose to use it together with egg killing agents to extend the duration of efficacy. Among the registered acaricides, only two varieties, i.e., Etoxazole and Spirodiclofen, are clearly indicates as ovicides, and their quantities are relatively rare. The scarcity of varieties and the increase in insecticide resistance have also led to an increasing demand for new effective double-kill type acaricides for both mites and mite eggs. At the same time, due to small molecular acaricides with multiple functions are scarce, when farmers apply them in the field, they usually need to mix and apply multiple agents such as regulators, foliar nutrition, bactericides, and egg killing agents simultaneously. The barrel mixing application of multiple chemical agents with different mechanisms of action and dosage forms will not only easily cause antagonistic effects among agents, but also easily damage the stability of aqueous solution of preparations, and phenomena such as precipitation, flocculation, stratification, etc., often occur, seriously affecting the field use of agents. Meanwhile, the mixed application of a large number of agents can easily lead to further increase in target resistance and adverse effects of pesticide residues on the environment.

Therefore, the development of novel and efficient insecticides and ovicides with novel structure and unique mechanisms of action is the key for prevention and control of agricultural pests.

### SUMMARY

The purpose of the present disclosure is to provide an insecticidal compound having a chiral isomeric sulfite, the absolute configurations of which can be prepared and isolated. One or more configurations of the compound can solve the problems existing in the prior art and achieve good inhibitory and killing effects on pest eggs.

Inventors have accidentally discovered that some of the compounds have low insecticidal activity (less than 75% reduction in insect population), but show excellent inhibitory activity on pest eggs when the insecticidal activity of sulfite compounds was studied.

The present disclosure provides a chiral isomeric sulfite compound having a structure as shown in formula (A) or a mesomer, a racemate, a stereisomer and a pharmaceutically acceptable salt thereof:

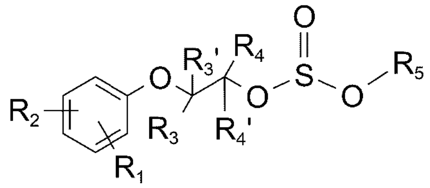

(A)

wherein, $R_1$ and $R_2$ are independently selected from hydrogen, halogen, a substituted or unsubstituted $C_1$-$C_{10}$ alkyl, a substituted or unsubstituted $C_{1-10}$ alkoxy, a $C_2$-$C_{10}$ alkoxycarbonyl, a $C_2$-$C_{10}$ alkylcarbonyl, and a $C_1$-$C_{10}$ carbonyl;
$R_3$, $R_3$', $R_4$, and $R_4$' are each independently selected from hydrogen, $C_1$-$C_5$ alkyl, $C_1$-$C_5$ alkenyl; or, $R_3$, $R_3$', $R_4$, and $R_4$' together with the carbon atom to which they are attached form a five-membered heterocycloalkyl;
$R_5$ is selected from halogen, and a substituted or unsubstituted $C_1$-$C_{10}$ alkyl.

Further, the structures of the compounds are selected from the group consisting of one of the following:

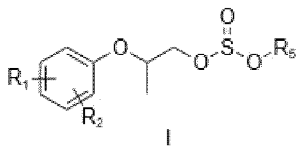

I

or

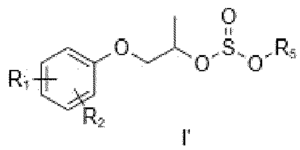

I'

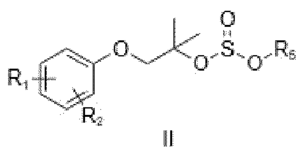

II

or

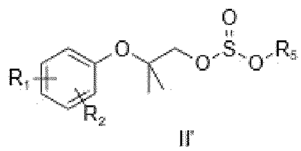

II'

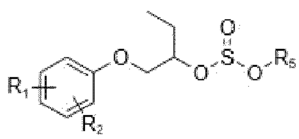

III

or

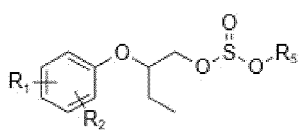

III'

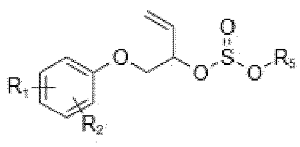

IV

or

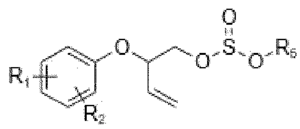

IV'

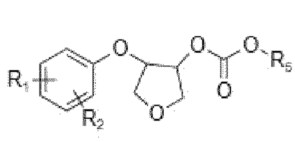

V

or

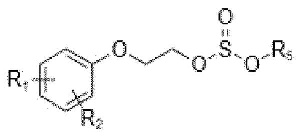

VI

.

In formulas (A) and I-VI, further, the $C_1$-$C_5$ alkyl is methyl and ethyl.
In formulas (A) and I-VI, further, the $C_1$-$C_5$ alkenyl is selected from ethenyl.
In formulas (A) and I-VI, further, the five-membered heterocycloalkyl is selected from

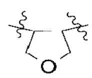

.

Further, the $R_5$ is selected from fluoroethyl, bromoethyl, chloroethyl, 2,2-difluoroethyl and 2,2-dichloroethyl; $R_1$ and $R_2$ are independently selected from H, F, Cl, or Br.

Further, $R_5$ is $-CH_2CH_2F$; $R_1$ and $R_2$ are Cl.

Further, the compound is selected from the following compounds:

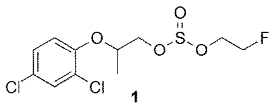
1

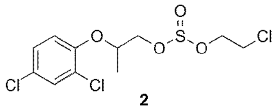
2

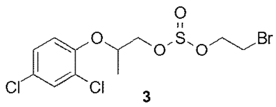
3

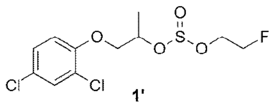
1'

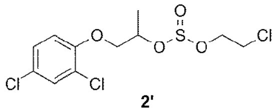
2'

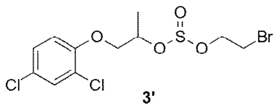
3'

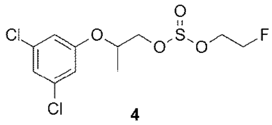
4

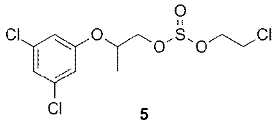
5

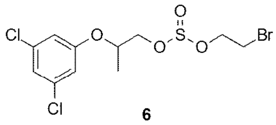
6

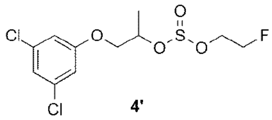
4'

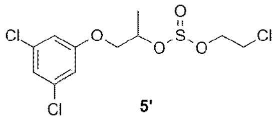
5'

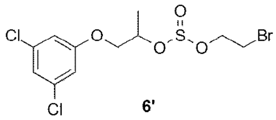
6'

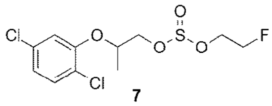
7

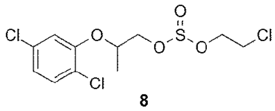
8

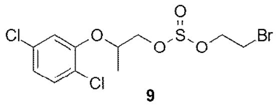
9

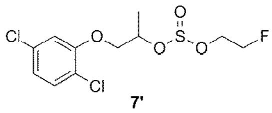
7'

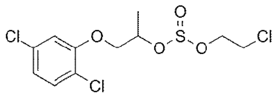
8'

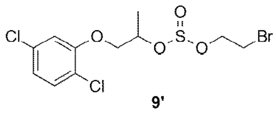
9'

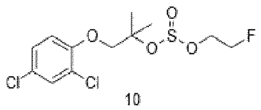
10

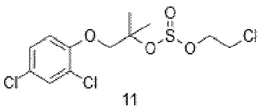
11

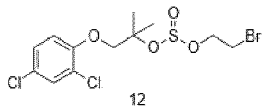
12

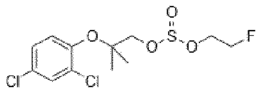
10'

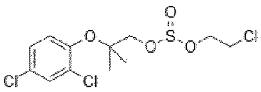
11'

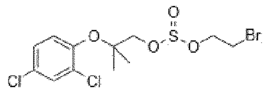
12'

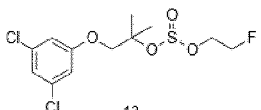
13

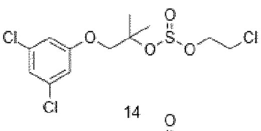
14

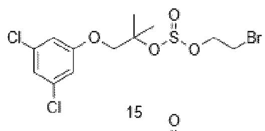
15

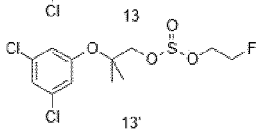
13'

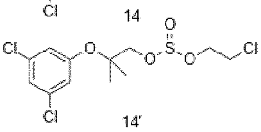
14'

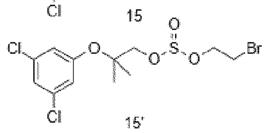
15'

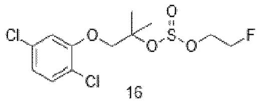
16
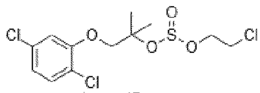
17
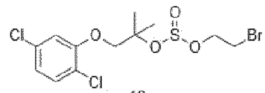
18

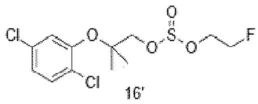
16'
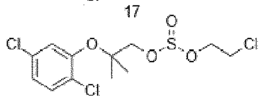
17'
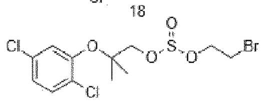
18'

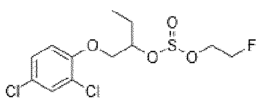
19
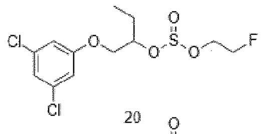
20
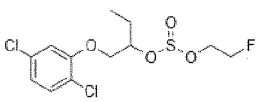
21

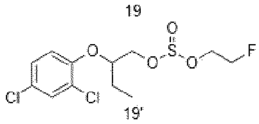
19'
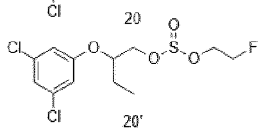
20'
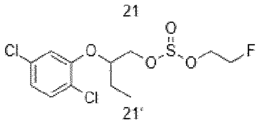
21'

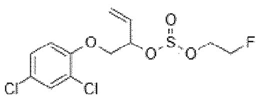
22
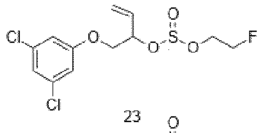
23
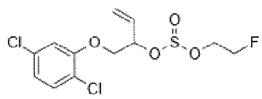
24

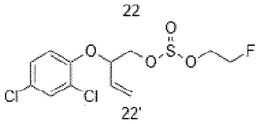
22'
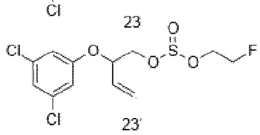
23'
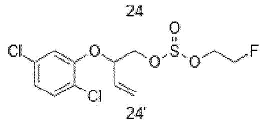
24'
.

Hereinbefore, unless otherwise specified, the term "substituted" means that the mentioned groups may be substituted by one or more additional groups, which are each independently selected from the group consisting of alkyl, cycloalkyl, aryl, carboxyl, heteroaryl, heterocycloalkyl, hydroxyl, alkoxy, alkylthio, aryloxy, O=, guanidino, cyano, nitro, acyl, halogen, haloalkyl, amino, etc.

The structures of the compounds in the present disclosure can be confirmed by conventional methods well-known to those skilled in the art. If the present disclosure involves the absolute configuration of a compound, the absolute configuration can be confirmed by conventional technical means in the art. According to the literature (Absolute configuration of glycosyl sulfoxides, Tetrahedron: Asymmetry, Volume 21, Issue 15, 2010, Pages 1830-1832). If the lone pair electrons of the group and sulfur are on the same side, the group is shielded, the chemical shift will decrease and move towards high fields. If the group is on the same side as the oxygen atom, the group is deshielded and the chemical shift will increase and move towards low fields. Based on the comparison and analysis of the 1H NMR spectroscopy of chirally isolated compounds and the testing of their optical activity, the absolute configurations of which can be determined.

The numbering of the above compounds is only for the convenience of subsequent explanation.

The present disclosure provides a method of controlling and/or killing pest eggs and/ or killing germs, including applying the above compounds onto the pest eggs and/or germs.

Further, when the compound is used for controlling and/or killing pest eggs and/or killing germs, it is selected from one of the following compounds, including mixtures of multiple configurations thereof:

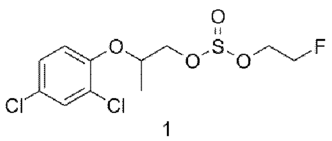
1
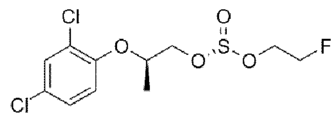
1-(R,R)
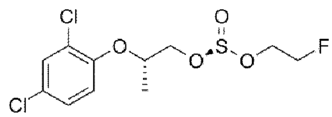
1-(S,S)

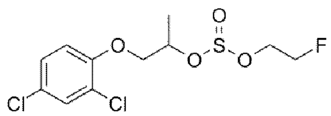
1'
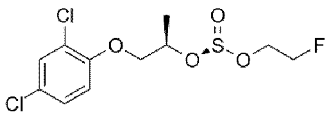
1'-(R,S)
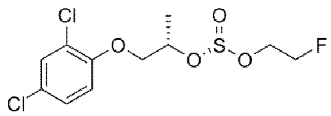
1'-(S,R)

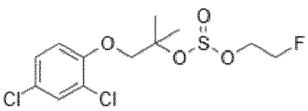
10
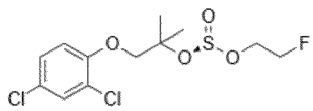
S-10
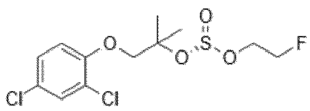
R-10
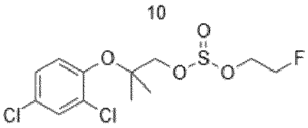
10'
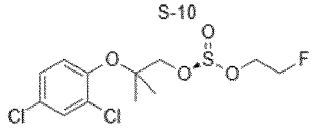
S-10'
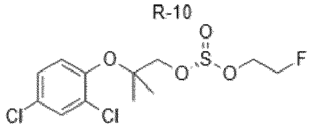
R-10'

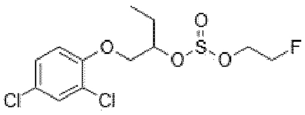
19
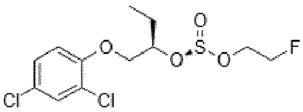
19-(R,S)
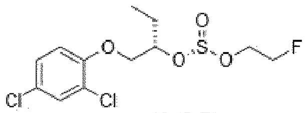
19-(S,R)
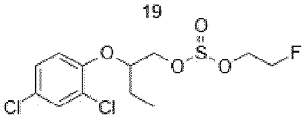
19'
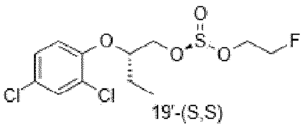
19'-(S,S)
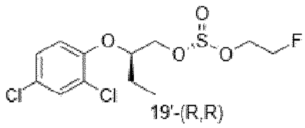
19'-(R,R)

The intermediate compounds of the present disclosure can be prepared through various synthesis methods well-known to those skilled in the art, including the specific embodiments listed herein, the embodiments formed by combining the specific embodiments with other chemical synthesis methods, and equivalent substitutions well-known to those skilled in the art, wherein the preferred embodiments include but are not limited to the embodiments of the present disclosure.

The chemical reactions in the specific embodiments of the present disclosure are carried out in suitable solvents, which must be compatible with the chemical transformations of the present disclosure and the required reagents and materials. In order to obtain the compounds of the present disclosure, sometimes it is necessary for those skilled in the art to modify or select the synthesis steps or reaction procedures based on the existing embodiments.

The term "pest" as used in this application refers to organisms that adversely affect the hosts (e.g., plants or animals such as mammals) by parasitizing, damaging, attacking, competing with them for nutrients, or infecting them.

Without specific limitations, pests include arthropods (including insects and spiders), as well as phloem-sucking pests and biting pests (such as bedbugs, mites, ticks, ants, lice, cockroaches, thrips, etc.).

Without specific limitations, the pest eggs are originated from insects of *Thysanoptera, Hemiptera, Lepidoptera, Coleoptera,* animals of *Acarina, Tetranychidae, Tenuipalpidae, Eriophyidae, Tarsonemidae, Pyemotidae, Penthaleidae* or *Cheyetidae;* and the germs include fungi and bacteria.

The *Thysanoptera* belongs to the *insecta,* insects of this order are commonly known as *"thrips".* They are small insects with slender bodies, are usually yellow brown or black in color, and have well-developed eyes, phloem-sucking mouthparts, and asymmetrical left and right; the wings are narrow and elongated, with a few or no wing veins, and the wing edges are prolate, with some long or short hairs; there are also species without wings and with only remnants left; they lack cerci; generally, they absorb juice from plants, which can harm cereals, cottons, tobaccos, etc. Some of them can spread plant viruses and become pests. Thrips are divided into *Terebrantia* and *Tubulifera. Terebrantia* includes: *Aeolothripoidea(Aeolothripidae, Orothripidae, Melanthripidae, Dactuliothripidae, Franklinothripidae), Merothripoidea (Aeolothripoidea), Thripoidea (Heterothripidae, Hemithripidae, Ceratothripidae, Panchaetothripidae, Thripidae ) ; wherein,*

*Thripidae is the largest and most important family in this order, with 33 genera and* approximately 200 species known in this family, such as *Frankliniella intonsa, Thrips tabaci Lindeman, Taeniothrips distalis Karny, Stenchaetothrips biformis, Thrips hawaiiensis Morgan, Thrips palmi Karny, Frankliniella occidentalis, Thrips japonicus Bagnall, Thrips serratus Kobus, Frankliniella tenuicornis Uzel, Scirtothrips dorsalis Hood, Heliothrips haemorrhoidalis Bouche, Scirtothrips dorsalis Hood, Scolothrips sexmaculatus Pergande,* etc., which are common species in our country. *Tubulifer*$\alpha$ include: *Phlaeothripoidea(Pypothripidae, Ecacanthothripidae, Eupatithripidae, Phlaeothripidae, Chirothripoididae, Hystricothripidae, Idolothripidae, Megathripidae), Urothripoidea* (*Urothripidae*). The parentheses after the superfamily indicate the subfamilies under the superfamily. Similarly, all others involving the superfamily hereinafter are expressed with this method.

The *Hemiptera* belongs to the *insecta,* has slightly flat and hard body, and has phloem-sucking mouthpart, filamentous or rod-shaped antennae, two or none ocelli, well-developed pronotum, ,and a mostly triangular scutellum; the forewings are hemelytra, and the hindwings are membranous; some species have degenerated or no wings; most species have scent glands; the end of dactylus often has tarsal claws, and there are claw pads under the claws; the abdomen has 9-11 segments, usually 10 segments; no cerci; named after the hemelytron of the anterior wings. The *Hemiptera* is divided into *Auchenorrhyncha* and *Sternorrhyncha;* the *Auchenorrhyncha* includes *Cicadiodea* (*Cicadidae, Membracidae, Machearotidae, Cercopidae, Cicadellidae)* and *Fulgoroidea* (*Tettigometridae, Delphacidae, Fulgoridae, Eurybrachydi-*

*dae, Cixiidae, Meenoplidae, Dictyopharidae, Achilidae, Tropiduchidae, Derbidae, Lophopidae, Issidae, Flatidae, Ricaniidae);* the *Sternorrhyncha* includes *Psylloidea* (*Psyllidae*), *Aleyrodoidea (Aleyrodidae), Aphidoidea* (*Adelgidae, Phylloxeridae, Pemphigidae, Aphididae )* and *Coccoidea (Margarodidae, Ortheziidae, Kerridae, Kermidae, Dactylopiidae, Pseudococcidae, Asterolecaniidae, Coccidae,* and *Diaspididae).*

The *Lepidoptera* belongs to the *insecta,* and has extremely wide distribution range, with tropical species being the most abundant; the vast majority of larvae pose a threat to various cultivated plants, with larger ones often consuming their leaves or boring their branches; smaller ones often rolling leaves, hanging on leaves, knotting sheaths, spinning silks and webs, or burrowing into plant tissues for feeding. Imagoes often supplement their nutrition with nectar or their mouthparts degrade and no longer feed. the *Lepidoptera* includes *Zeugloptera* (*Micropterygidae*)*, Monotrysia* (*Eriocraniidea, Hepialoidea, Stigmelloidea, Incurvarioidea* ) and *Ditrhysia* (*Tinaeoidea, Cossoidea, Psychoidea, Castnioidea, Tortricoidea, Pyraloidea, Bombycoidea, Calliduloidea, Geometroidea, Sphingoidea, Noctuoidea, Hesperioidea* and Papilionoidea).

The *Coleoptera* is the largest and most widely distributed order in the *insecta* and even in the *animalia.* It is divided into *Adephaga, Polyphaga, Rhynchophora;* the *Adephaga* includes: *Caraboidea (Cicindelidae, Carabidae, Amphizoidae, Omophronidae, Hygrobiidae, Haliplidae, Dytiscidae), Gyrinoidea (Gyrinidae), Paussoidea (Paussidae), Cupesoidea (Cupesidae), Rhysodoidea (Rhysodidae);* the *Polyphaga* includes: *Hydrophiloidea (Hydrophilidae), Staphylinoidea (Silphidae, Leiodidae, Clambidae, Scydmaenidae, Orthoperidae, Phaenocephalidae, Discolomidae, Platypsyllidae), Cantharoidea (Lycidae, Lampyridae, Cantharidae, Drilidae, Malachiidae, Phloeophilidae, Prionoceridae, Dasytidae), Lymexyloidea (Lymexylidae, Atractoceridae), Elateroidea (Rhipiceridae, Cebrionidae, Elateridae, Eucnemidae, Throscidae), Dryopoidea (Psephenidae, Dryopidae, Helmidae, Georyssidae, Heteroceridae), Dascilloidea (Dascillidae), Tenebrionoidea (Alleculidae, Tenebrionidae), Ptinidae (Lyctidae, Bostrychidae, Anobiidae, Ptinidae), Scarabaeoidea (Scarabaeidae, Aegialiidae, Aphodiidae, Ochodaeidae, Geotrupidae, Trogidae, Melolonthidae, Rutelidae, Dynastidae, Cetoniidae, Trichiidae, Passalidae), Cerambycoidea (Prionidae, Cerambycidae, Lamiidae, Sagridae), Brentoidea (Brentidae), Curculionoidea(Anthribidae, Aglycyderidae, Proterhiniidae, Cyladidae, Curculionidae); the Rhynchophora* includes*:Curculionoidea (Anthribidae, Aglycyderidae, Proterhiniidae, Cyladidae, Curculionidae).* Common insects (commonly known as): *Harmonia axyridis, Cerambycidae, Coccinellidae, Lampyridae, Scarabaeidae, Mylabris phalerata, Allomyrina dichotoma, Buprestidae, Melyridae, Scarabaeidae, Lucanidae, Elateridae, Dytiscidae, and Sitophilus oryzae.*

The "mites" as used in this application mainly include agricultural pest mites, most of which belong to *Tetranychidae, Tenuipalpidae, Eriophyidae, Tarsonemidae, Pyemotidαe, Penthaleidae* and *Cheyetidae* of *Acachnida.*

The *Tetranychidae* is divided into *Oligonychus* (such as *Oligonychus baipisongis, Oligonychus karamatus, Oligonychus rubicundus,* etc.), *Eotetranychus* (such as *Eotetranychus albus, Eotetranychus bailae, Eotetranychus camelliae,* etc.), *Tetranychus* (such as *Tetranychus neocaledonicus, Tetranychus phaselus, Tetranychus urticae, Tetranychus cinnabarinus*), *Schizotetranychus* (such as *Schizotetranychus baltazarae, Schizotetranychus bambusae, Schizotetranychus elongatus,* etc.), *Mixonychus* (such as *Mixonychus (Bakerina) aestiva, Mixonychus (Mixonychus) ganjuis, Mixonychus (Bakerina) murrayae,* etc.), *Panonychus* (such as *Panonychus citri, Panonychus caglei, Panonychus ulmi,* etc.), *Allonychus* (such as *Allonychus bambusae, Allonychus wuyinicus), Stigmaeopsis* (such as *Stigmaeopsis celarius, Stigmaeopsis nanjingensis), Mononychellus* (such as *Mononychellus georgicus*), *Acanthonychus* (such as *Acanthonychus jiangfengensis), Amphitetranychus* (such as *Amphitetranychus viennensis*), *Sonotetranychus* (such as *Sonotetranychus neosalix*), *Xinella* (such as *Xinella huangshanensis*), *Yunonychus* (such as *Yunonychus daliensis), Neotetranychus* (such as *Neotetranychus lek*), *Eurytetranychus* (such as *Eurytetranychus glycyrrhizae, Eurytetranychus wuyishanensis*), *Aponychus* (such as *Aponychus aequilibris, Aponychus corpuzae), Eutetranychus* (such as *Eutetranychus orientalis, Eutetranychus xianensis*), *Stylophoronychus* (such as *Stylophoronychus baghensis), Eurytetranychoides* (such as *Eurytetranychoides japonicus*), *Tenuipalpoides* (such as *Tenuipalpoides hastata, Tenuipalpoides zizyphus), Bryobia* (such as *Bryobia borealis, Bryobia exserta*), *Sinobryobia* (such as *Sinobryobia chinensis*)*, Petrobia* (such as *Petrobia (Petrobia) xinjiangensis, Petrobia* (*Tetranychina*) *zachvatkini,* etc.), *Tetranycopsis* (such as *Tetranycopsis hystriciformis, Tetranycopsis spiraeae,* etc.), *Aplonobia* (such as *Aplonobia alkalisalinae), Mesobryobia* (such as *Mesobryobia terpoghossiani*), *Dolichonobia* (*Dolichonobia altaiensis*).

Further, the pest eggs are originated from *Frankliniella intonsa, Thrips tabaci Lindeman, Taeniothrips distalis Karn, Stenchaeotothrips biformis, Thrips hawaiiensis Morgan, Thrips palmi Karny, Frankliniella occidentalis, Thrips japonicus Bagnall, Thrips serratus Kobus, Frankliniella tenuicornis Uzel, Scirtothrips dorsalis Hood, Heliothrips haemorrhoidalis Bouche, Scirtothrips dorsalis Hood, Scolothrips sexmaculatus Pergande, Cnaphalocrocis medinalis, Spodoptera exigua*, *Spodoptera litura, Carposina sasakii, Helicoverpa armigera, Plutella xylostella, Diaphania indica, Maruca testulalis Geyer, Bemisia tabaci Gennadius, Trialeurodes vaporariorum, Aleurocanthus spiniferus, Dialeurodes citri Ashm,* Bemisia myricae Kuwana, *Aleurocybotus indicus, Aleurodicus dispersus, Oligonychus baipisongis, Oligonychus karamatus, Oligonychus rubicundus, Cerambycidae), Coccinellidae, Lampyridae, Scarabaeidae, Mylabris phalerata, Allomyrina dichotoma, Buprestidae, Melyridae,* Scarabaeidae, Lucanidae, *Elateridae, Dytiscidae, Sitophilus oryzae, Harmonia axyridis, Eotetranychus albus, Eotetranychus bailae, Eotetranychus camelliae, Tetranychus neocaledonicus, Tetranychus phaselus, Tetranychus urticae, Tetranychus cinnabarinus, Schizotetranychus baltazarae, Schizotetrany-*

*chus bambusae, Schizotetranychus elongatus, Mixonychus (Bakerina) aestiva, Mixonychus (Mixonychus) ganjuis, Panonychus citri, Panonychus caglei, Allonychus bambusae, Allonychus wuyinicus, Stigmaeopsis celarius, Mononychellus georgicus, Acanthonychusjiangfengensis, (Amphitetranychus viennensis.*

Among them, the germs are selected from fungi.

In a specific embodiment of the present disclosure, the fungus is selected from Magnaporthe grisea. The Magnaporthe oryzae causes rice blast, which can damage seedlings, leaves, panicles, nodes, etc.

The term "control" as used in the present disclosure includes but is not limited to arbitrary killing of pest eggs, hatching and regulating, inhibition/interference of pest egg activity, prevention of hatching, etc.; the phrase "preventing hatching" refers to preventing or delaying the hatching of larvae from eggs.

The term "killing" as used in the present disclosure refers to the permanent loss of the ability of the pest eggs to grow and hatch.

The term "killing germs" as used in the present disclosure refers to directly killing or inhibiting the growth of plant pathogens, the pathogenic microorganism of which includes fungi and bacteria; the killing germs includes protective killing germs and systemic killing germs. The protective killing germs involves direct contacting with pathogenic bacteria outside or on the body surface of plants, so as to kill or inhibit pathogenic bacteria, so that they cannot enter the plants, thereby protecting the plants from the harm of pathogenic bacteria; and the systemic killing germs involves being absorbed by plants and transmitted to the sites infected by pathogenic bacteria within the body to eliminate the pathogenic bacteria.

In the present disclosure, when used, the sulfite compound is prepared into an agricultural product, which further includes one or more accessories such as a dispersant, a wetting agent, a binder, a surfactant, a stabilizer and a solvent.

Suitable surfactants can be selected by those skilled in the art according to actual use demands. Examples of surfactants that can be used in some embodiments of the present disclosure include, but are not limited to, ethoxylated castor oil, sodium lauryl sulfate, saponin, ethoxylated alcohols, ethoxylated fatty esters, alkoxylated diols, ethoxylated fatty acids, carboxylated alcohols, carboxylic acids, fatty acids, ethoxylated alkylphenols, fatty esters, sodium dodecyl sulfide, other fatty acid-based surfactants, other natural or synthetic surfactants, and combinations thereof. In some embodiments, the surfactant is a non-ionic surfactant. In some embodiments, the surfactant is an ionic surfactant. The selection of appropriate surfactants depends on the relevant applications and usage conditions, and appropriate surfactants are known to those skilled in the art.

In the present disclosure, the dosage forms include but are not limited to missible oil, soluble powder, soluble granule, solution, dispersible liquid, emulsion in water, microemulsion, microcapsule suspension concentrate, seed treatment liquid, aerosol, etc.

Missible oil is one type of pesticide formulations, which is a liquid formed by dissolving high concentrations of active ingredients in a solvent and then adding an emulsifier. Generally, after the missible oil is diluted with a large amount of water to form a stable emulsion, it can be dispersed with a sprayer, or it can be sprayed in a low volume or even ultra-low volume, or it can be used directly or diluted with water before spraying.

Wettable powder is a very fine dry agent obtained by mixing and crushing technical materials, fillers, surfactants, and other adjuvants, etc. together.

Suspension concentrate refers to a preparation formed by uniformly dispersing solid technical materials in water in particulates of less than 4 microns, with the international code SC. The particle size of which is fine, generally ranging from 0.1-3 $\mu$m, and the suspension rate is high. Suspension concentrate is divided into two types, i.e., water suspension concentrate and oil suspension concentrate. Water suspension concentrate uses water as the suspension medium, while oil suspension concentrate uses oils as the suspension medium and does not contain water. The commonly used oils are vegetable oils, such as corn oil, and rapeseed oil, etc. Suspension concentrate needs no organic solvents at all and is a good dosage form for processing solid technical materials. Suspension concentrate is a mixture of solid powder and liquid suspended in water, which needs to be shaken well before use, then diluted with water, and sprayed. Suspension concentrates are easy to carry and dilute, can be evenly sprayed, and have good adhesion and duration of efficacy.

Powder refers to the technical material powder or the powder prepared by adding a certain diluent. It can be directly sprayed with a simple powder sprayer, with high work efficiency, low crop adhesion, less amount of residues, and less likely to cause insecticide damage.

Granule, also known as particle, refers to a solid dosage form obtained by mixing technical materials with adjuvants such as carriers, adhesives, dispersants, wetting agents, stabilizers, etc., for granulation Its performance requirements mainly include fineness, uniformity, storage stability, hardness, disintegration, etc. Granules have the largest particle size among solid dosage forms, with a diameter of 300-1700 um. They have the advantages of simple use, small outdiffusion, and long-lasting pesticide effect.

Water aqua is a solution of technical materials, and the agent is uniformly dispersed in water in the form of ions or molecules. The concentration of the agent depends on the solubility in water of the technical materials, which is generally its maximum solubility, and it is diluted with water when used.

In the present disclosure, the sulfite compound can be used in combination with the majority of commercially available agricultural preparations such as insecticides, acaricides, bactericides, etc., to achieve synergistic effects.

Further, when the sulfite compound is used to control and/or kill pest eggs of pests and/or killing germs, the concentration of the sulfite compound should be not less than 0.1 ppm. Further more, the concentration of the sulfite compound is not less than 1 ppm.

Wherein, the sulfite compound is applied in a concentration of 0.1-10000 ppm, or the concentration may also be 0.1-500 ppm, 0.1-200 ppm, 0.1-100 ppm, 0.1-50 ppm, 1-500 ppm, 1-200 ppm, 1-100 ppm, 1-50 ppm, 1-10 ppm, 1-5 ppm, 2-200 ppm, 2-100 ppm, 2-50 ppm, 2-10 ppm, 3-200 ppm, 3-100 ppm, 3-50 ppm, 3-10 ppm, 4-200 ppm, 4-100 ppm, 4-50 ppm, 4-10 ppm, and 10-1000 ppm. Specifically, it can be selected from but not limited to: 0.1 ppm, 0.2 ppm, 0.3 ppm, 0.4 ppm, 0.5 ppm, 0.6 ppm, 0.7 ppm, 0.8 ppm, 0.9 ppm, 1 ppm, 1.1 ppm, 1.2 ppm, 1.3 ppm, 1.4 ppm, 1.5 ppm, 1.6 ppm, 1.7 ppm, 1.8 ppm, 1.9 ppm, 2.0 ppm, 2.5 ppm, 3 ppm, 3.5 ppm, 4 ppm, 4.5 ppm, 5 ppm, 5.5 ppm, 6 ppm, 6.5 ppm, 7 ppm, 7.5 ppm, 8 ppm, 8.5 ppm, 9 ppm, 9.5 ppm, 10 ppm, 11 ppm, 12 ppm, 13 ppm, 14 ppm, 15 ppm, 20 ppm, 25 ppm, 30 ppm, 35 ppm, 40 ppm, 45 ppm, 50 ppm, and 100 ppm, and so on.

Further more, when the sulfite compound is used to control and/or kill pest eggs, the sulfite compound is applied in a concentration of 0.1-500 ppm; and when the sulfite compound is used for killing germs, the sulfite compound is applied in a concentration of 10-1000 ppm.

The present disclosure also provides a pesticide composition, wherein the compound of formula (I) is used as the active substance.

In the present disclosure, the pesticide composition may also include a dispersant, a wetting agent, a binder, a surfactant, a stabilizer, a solvent, etc.

In the present disclosure, the pesticide composition may also be compounded with other products, including but not limited to one or more of other insecticides, acaricides, bactericides, herbicides, plant growth regulators or fertilizers, and compounds with equivalent functions that have not yet been commercialized, thereby generating additional advantages and effects. For example, other insecticides can include Flupyradifurone, Decamethrin, Ethiprole, Tetraniliprole, Imidacloprid, Spirotetramat, Spirodiclofen, Afidopyropen, Chlorfenapyr, Alphacypermethrin, Broflanilide, Cyetpyrafen, Lambdacyhalothrin, Pymetrozine, Thiamethoxam, Lufenuron, Avermectin, Chlorantraniliprole, Bifenthrin, Cyantraniliprole, Cyflumetofen, Spinetoram, Triflumezopyrim, Sulfoxaflor, Pirimiphos-methyl, Indoxacarb, Dinotefuran, Temephos, Hydramethylnon, Permethrin, Hexaflumuron, Bisulflufen.

In one embodiment of the present disclosure, the pesticide composition further comprises a mixture of ginger rhizome extract and Kaempferia galanga L rhizome extract in a ratio of 7:3, wherein the ginger rhizome extract is obtained by extracting the ginger rhizome with ethanol:ethyl acetate=1-4:1; and the Kaempferia galanga L rhizome extract is a volatile oil from Kaempferia galanga L rhizome. The compounding ratio of the above mixture of ginger and Kaempferia galanga L to the compound of formula (A) may be (200-500):(0.1-1). Experimental studies have shown that the combination of the two may have a certain synergistic effect and can reduce the concentration of the active ingredients.

The term "including" or "containing" as used in this application shall be interpreted in its open-ended, meaning that it is specified that there are specified features, elements, steps, or components as mentioned, but it does not exclude the presence or addition of further features, elements, steps, or components.

In some embodiments, any of the above compositions are applied outdoors, or to the interior or exterior of plants or agricultural areas and/or buildings. In some embodiments, any of the above compositions are applied to the surface of homes, dwellings, or buildings. In some embodiments, any of the above compositions are applied to mattresses, sheets, fabrics, travel bags/suitcases, carpets, painted or unpainted hard surfaces, wood, floorings, furnitures, and/or buildings.

In some embodiments, any of the above compositions are prepared in deliverable forms suitable for specific applications. These deliverable forms include but are not limited to liquid, emulsion, solid, wax, dust, fumigant, aqueous suspension, oil dispersion, paste, powder, dust, emulsifiable concentrate, aerosol spray, wood sealer, varnish, wood treatment or furniture oil, cleaner, dry wall mixture, incense candle, joint filling composition, crack and fissure filler, sealant, and mattress and bedspread treatment. Suitable deliverable forms can be selected and formulated by those skilled in the art using methods known in the art. In different usage scenarios, the above-mentioned compositions can be applied in various ways, they can be used directly, diluted before use, and used in concentrated forms. In addition, they can also be used for the following purposes: protective oil for wood or furniture, laundry cleaner, gel or cream that can be applied in the target area, oil-based emulsion, part of dry wall materials used as a mixture of dust, filler or other sealing materials used to fill cracks or gaps, foam, part of joint filling materials, incense mist or candles, aerosol or spray insecticides, and treatment agent for mattresses or bedspreads. In some cases, these mixtures can be used in household or commercial environments to combat pest eggs and fungi in a dispersed form. In addition, they can also be used in agricultural or other outdoor environments to control pest eggs and fungi.

The "solvent" used in the above products or compositions can be selected from water, ketones, alcohols, aldehydes, ethers, esters, or carboxylic acids, and can include non-aromatic ketones, non-aromatic alcohols, non-aromatic aldehydes, non-aromatic esters, non-aromatic carboxylic acids, aromatic alcohols, aryl-alkyl alcohols, aryl aldehydes, aryl-alkyl ketones, aryl-aryl ketones, aryl carboxylic acids, aryl-alkyl esters, aryl-alkyl ethers, aryl-aryl ethers, and/or combinations thereof.

In some embodiments, the solvents include ethanol, isopropanol, benzyl alcohol, acetone, acetophenone, water,

citric acid, lactic acid, glycerol, castor oil, benzoic acid, carbonic acid, ethoxylated alcohols, ethoxylated amides, glycerides, butanol, 1-propanol, hexanol, other alcohols, dimethyl ether, polyethylene glycol, etc.

The beneficial effects of the present disclosure are as follows: the present disclosure provides the application of the chiral, isomeric sulfite compounds in inhibiting pest eggs. The chiral, isomeric sulfite compounds have a strong inhibitory effect on eggs, especially on the eggs of various thrips, mites, and Aleyrodidae in *Thysanoptera*; the compounds of the structure can be used as insecticides or ovicides, and have high research value of agrochemical, having broad application prospects in the field of agricultural pharmacy.

## DESCRIPTION OF DRAWINGS

Figure 1 shows the chromatogram of separation of a mixture of compound 1 and compound 1' using an IG column;
Figure 2 shows the chromatogram of separation of R configuration of compound 1 and compound 1' using an IG column;
Figure 3 shows the chromatogram of separation of S configuration of compound 1 and compound 1' using an IG column;
Figure 4 shows the chromatogram of a first peak separated from R configuration in an IG column;
Figure 5 shows the chromatogram of a second peak separated from R configuration in an IG column;
Figure 6 shows the chromatogram of a third peak separated from R configuration in an IG column;
Figure 7 shows the chromatogram of a fourth peak separated from R configuration in an IG column;
Figure 8 shows the chromatogram of a first peak separated from S configuration in an IG column;
Figure 9 shows the chromatogram of a second peak separated from S configuration in an IG column;
Figure 10 shows the chromatogram of a third peak separated from S configuration in an IG column; and
Figure 11 shows the chromatographic peak when separated using an AS column for the first peak obtained after the separation of S configuration of compound 1 and compound 1' on an IG column.

## DETAILED DESCRIPTION

In order to more clearly clarify the purposes, technical solutions and advantages of the present disclosure, the present disclosure will be further explained below in detail in combination with the embodiments. It should be understood that the specific embodiments as described herein are only used to explain the present disclosure and are not intended to limit the present disclosure. In addition, if not explicitly stated, all reagents, raw materials, and other experimental supplies used in the following examples are commercially available or can be synthesized, cultured, or cultivated according to the methods described herein or known in the art. For experimental conditions not listed herein, they are also easily obtained by those skilled in the art.

When providing a numerical range, it is understood that the intermediate values between the upper and lower limits of the range (up to one tenth of the unit of the lower limit, unless otherwise explicitly indicated in the context) and any other specified values or intermediate values within the specified range are covered in the embodiments of this application. The upper and lower limits of these smaller ranges can independently define smaller numerical ranges, and it will be understood that these smaller ranges are intended to be covered in the embodiments of this application, subjecting to any explicitly excluded limits within the specified range.

Unless otherwise limited, all technical and scientific terms used herein have the same meanings as those commonly understood by ordinary technical personnels in the art to which this application belongs. Any methods and materials similar or equivalent to those described herein can also be used in the practice or testing of the embodiments of this application.

### Example 1 Synthesis of Compounds S-1 and S-1'

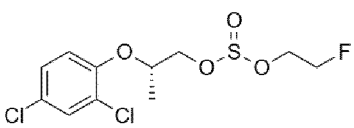

**S-1**

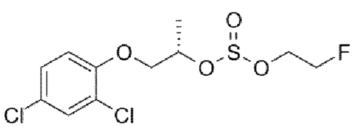

**S-1'**

Step 1:

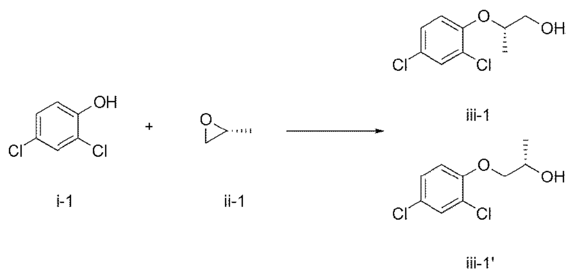

i-1 ii-1 iii-1 iii-1'

2,4-dichlorophenol (500 mg, 3.1 mmol), R-propylene oxide (356 mg, 6.1 mmol), DMF (12 mL), and cesium carbonate (4.0 g, 12.3 mmol) were added to a reaction flask, which was heated to 100°C, and refluxed, the reaction was monitored by TLC. When the reaction was completed, it was concentrated, the residue was dissolved in water (10 mL) and ethyl acetate (50 mL), then subjected to liquid seperation, the aqueous phase was extracted with ethyl acetate (50 mL × 2), the organic phases were combined, washed with saturated saline solution, dried over anhydrous sodium sulfate, filtered, concentrated, and subjected to column chromatography to obtain compounds iii-1, iii-1' (511 mg in total, colorless transparent oil).

Step 2:

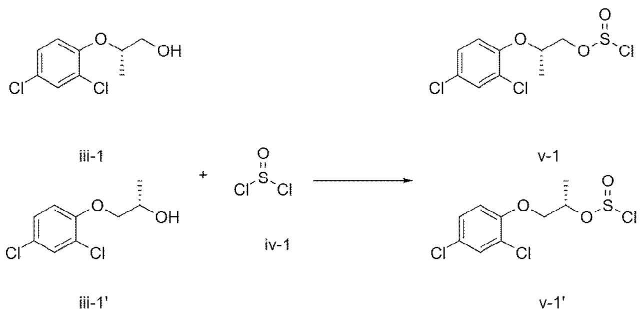

iii-1 iii-1' iv-1 v-1 v-1'

Thionyl chloride (415 mg, 3.5 mmol) was added into a reaction flask, and dissolved in 15 mL of dichloromethane, the reaction flask was transferred to an ice bath at 0°C and a mixture of compounds iii-1 and iii-1' (511 mg, 2.3 mmol) was added dropwise slowly therein while stirring. After the dropwise addition was completed, the reaction flask was warmed to room temperature and reacted for 10 hours. The reaction was monitored by TLC until it was completed, and the reaction solution was concentrated under reduced pressure to obtain a light yellow oil, which was the crude product of compounds v-1 and v-1', and was ready for later use.

Step 3:

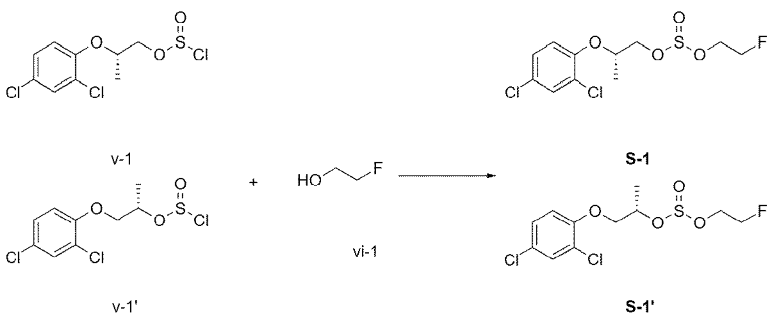

v-1 v-1' vi-1 **S-1** **S-1'**

Compound vi-1 (224 mg, 3.5 mmol) was added into a reaction flask, triethylamine (349 mg, 3.5 mmol) was added thereto, then the reaction flask was transferred to an ice bath at 0°C and a mixture of compounds v-1 and v-1' (690 mg, 2.3 mmol) was added dropwise slowly therein while stirring. After the dropwise addition was completed, the reaction flask was warmed to room temperature and reacted for 6 hours. The reaction was monitored by TLC until it was completed, then 100 mL of water was added to the reaction solution and extracted with dichloromethane (30 mL × 3), the organic phases were collected after washing with saturated saline solution, dried over anhydrous sodium sulfate, concentrated under reduced pressure, and purified by column chromatography to obtain a mixture of compounds S-1 and S-1'(501 mg, colorless transparent liquid).

**Example 2 Synthesis of Compounds R-1 and R-1'**

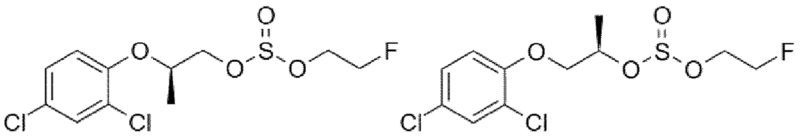

R-1 R-1'

The R-propylene oxide in step 1 of Example 1 was replaced with S-propylene oxide, and the reaction was carried out according to the synthesis method of Example 1 to obtain a mixture of compounds R-1 and R-1' (515 mg, colorless transparent liquid).

**Example 3 Synthesis of Compounds S-19 and S-19'**

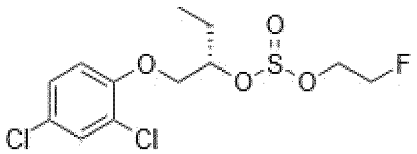

S-19

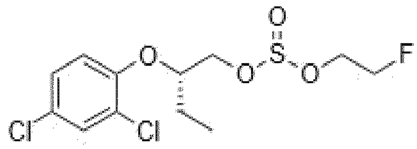

S-19'

Step 1:

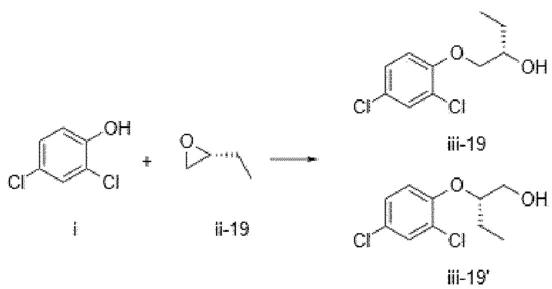

i ii-19 iii-19 iii-19'

According to the synthetic method in step 1 of Example 1, the R-propylene oxide was replaced with R-1,2-butylene oxide to obtain a mixture of the target compounds iii-19 and iii-19'.

Step 2 was the same as step 2 in Example 1.

Step 3 was the same as step 3 in Example 1.

The target compounds S-19 and S-19' (523 mg, colorless transparent liquid) were synthesized.

**Example 4 Synthesis of Compounds R-19 and R-19'**

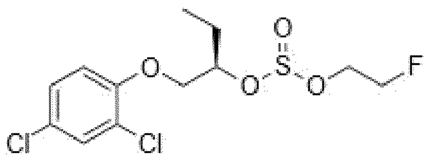

R-19

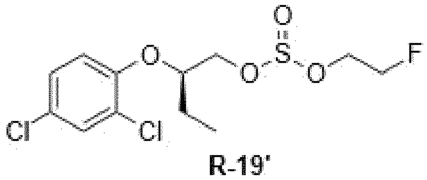

R-19'

The R-propylene oxide in step 1 of Example 1 was replaced with S-1,2-butylene oxide, and a mixture of compounds R-19 and R-19' (507 mg, colorless transparent liquid) was synthesized according to the synthetic method of Example 1.

**Example 5 Chiral Separation of Compounds S-1, S-1', R-1, R-1', Compound 10, Compound 10', S-19, S-19', R-19, and R-19'**

1. Experimental Method

1.1 Separation of Compounds

The sulfite compounds 1-9, 1'-9', 19-24, and 19'-24' were preliminarily judged to have two chiral centers. The chiral raw materials were used for synthesis and then the products were separated with Instrument: Shimadzu high-pressure liquid preparative chromatography, LC20AR; Chiral Column: CHIRALPAK® Positive Phase IG column, CHIRALPAK® Positive Phase AS column, 4.6 mm I.D.×250 mm, Diameter: 5 μm; Mobile Phase: n-hexane:isopropanol=95:5.

The S mixture of Compound 1 and compound 1' was separated into three components using an IG column, the R mixture of compound 1 and compound 1' was separated into four components using an IG column; and the separated seven components were further separated by an AS column, it was found that the first peak in the S configuration of compound 1 and compound 1' contained two components, which were further separated, and a total of eight components were obtained.

In the synthesis of compound 1, the reaction in the first step resulted in different methyl positions due to the different ring opening positions, resulting in two products, i.e., compounds iii-1 and iii-1', which can be separated by chiral preparative chromatography. Instrument: Shimadzu high-pressure liquid phase chromatography, LC20AR; Chiral Column: CHIRALPAK® Positive Phase IG column, 4.6 mm I.D.×250 mm, diameter: 5 μm; Mobile Phase: n-hexane: isopropanol=90:10. (Compounds iii-4 and iii-4', etc. were separated using the same conditions)

1.2 Determination of Absolute Configuration of Compounds

1.2.1 H NMR Spectroscopy of Compounds

According to the literature (Absolute configuration of glycosyl sulfoxides, Tetrahedron: Asymmetry, Volume 21, Issue 15,2010, Pages 180-1832, https://doi.org/10.1016/j.tetasy.2010.06.019.), if the lone pair electrons of the group and sulfur are on the same side, the group is shielded, the chemical shift will decrease and move towards high fields. If the group is on the same side as the oxygen atom, the group is unshielded and the chemical shift will increase and move towards low fields. Based on this result, the data analysis of H NMR spectroscopy of the separated compounds was carried out, and it is speculated that their absolute configuration are as follows:

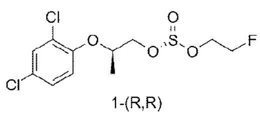

1-(R,R)

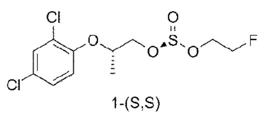

1-(S,S)

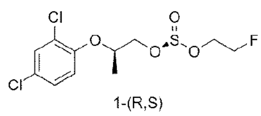

1-(R,S)

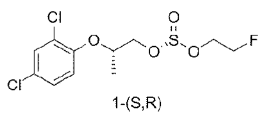

1-(S,R)

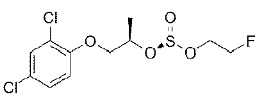

1'-(R,S)

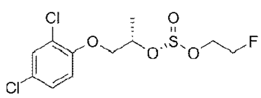

1'-(S,R)

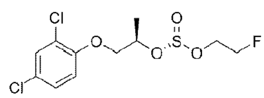

1'-(R,R)

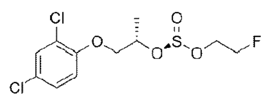

1'-(S,S)

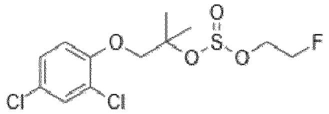
10
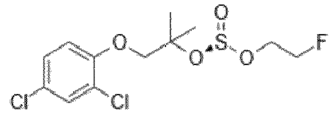
S-10
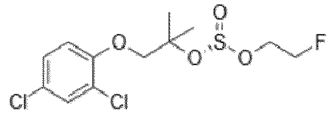
R-10

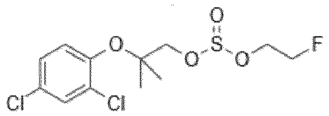
10'
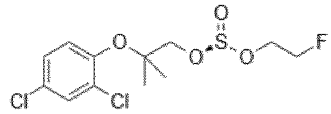
S-10'
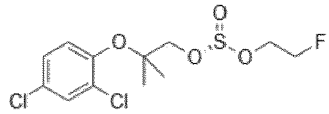
R-10'

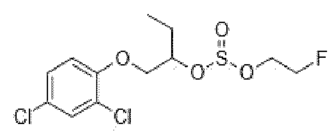
19
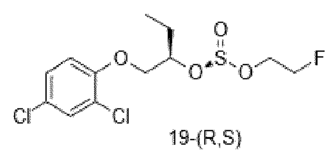
19-(R,S)
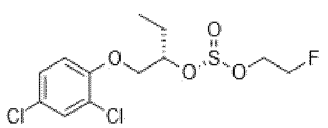
19-(S,R)

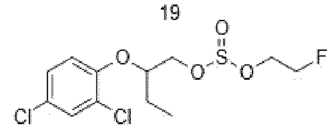
19'
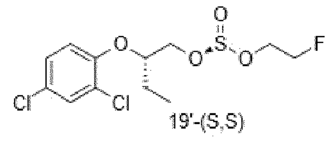
19'-(S,S)
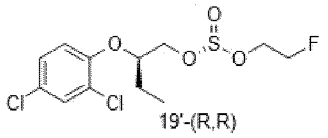
19'-(R,R)

**1-(R,R):** $^1$H NMR (400 MHz, $CDCl_3$) δ 7.31 *(d,* *J* = 2.5 Hz, 1H), 7.11 (dd, *J* = 8.8, 2.5 Hz, 1H), 6.76 (d, *J* = 8.8 Hz, 1H), 4.90 (td, *J* = 6.6, 4.1 Hz, 1H), 4.62 (dd, *J* = 5.1, 3.1 Hz, 1H), 4.53 - 4.45 (m, 1H), 4.37 - 4.10 (m, 2H), 3.98 (qd, *J* = 10.0, 5.4 Hz, 2H), 1.39 (d, *J* = 6.5 Hz, 3H) ppm.

**1-(R,S):** $^1$H NMR (400 MHz, $CDCl_3$) δ 7.31 (d, *J* = 2.5 Hz, 1H), 7.11 (dd, *J* = 8.8, 2.6 Hz, 1H), 6.85 (d, *J* = 8.8 Hz, 1H), 4.64 - 4.55 (m, 1H), 4.55 - 4.38 (m, 2H), 4.22 - 4.11 (m, 3H), 4.04 (dd, *J* = 11.2, 4.1 Hz, 1H), 1.32 (d, *J* = 6.3 Hz, 3H) ppm.

**1-(S,R):** $^1$H NMR (400 MHz, $CDCl_3$) δ 7.31 (d, *J* = 2.5 Hz, 1H), 7.11 (dd, *J* = 8.8, 2.6 Hz, 1H), 6.85 (d, *J* = 8.8 Hz, 1H), 4.64 63 - 4.55 (m, 1H), 4.55 - 4.38 (m, 2H), 4.24 - 4.11 (m, 3H), 4.04 (dd, *J* = 11.2, 4.1 Hz, 1H), 1.32 (d, *J* = 6.3 Hz, 3H) ppm.

**1-(S,S):** $^1$H NMR (400 MHz, $CDCl_3$) δ 7.31 (d, *J* = 2.5 Hz, 1H), 7.11 (dd, *J* = 8.8, 2.5 Hz, 1H), 6.76 (d, *J* = 8.8 Hz, 1H), 4.90 (td, *J* = 6.6, 4.1 Hz, 1H), 4.62 (dd, *J* = 5.1, 3.1 Hz, 1H), 4.56 - 4.45 (m, 2H), 4.37 - 4.10 (m, 2H), 3.98 (qd, *J* = 10.0, 5.4 Hz, 2H), 1.39 (d, *J* = 6.5 Hz, 3H) ppm..

**1'-(S,R):** $^1$H NMR (400 MHz, $CDCl_3$) δ 7.30 (d, *J* = 2.5 Hz, 1H), 7.11 (dd, *J* = 8.8, 2.5 Hz, 1H), 6.76 (d, *J* = 8.8 Hz, 1H), 4.88 (pd, *J* = 6.4, 4.4 Hz, 1H), 4.68 - 4.56 (m, 1H), 4.55 - 4.45 (m, 1H), 4.34 - 4.11 (m, 2H), 4.01 (dd, *J* = 10.0, 6.3 Hz, 1H), 3.93 (dd, *J* = 10.0, 4.3 Hz, 1H), 1.43 (d, *J* = 6.5 Hz, 3H) ppm.

**1'-(S,S):** $^1$H NMR (400 MHz, $CDCl_3$) δ 7.31 (d, *J* = 2.5 Hz, 1H), 7.11 (dd, *J* = 8.8, 2.5 Hz, 1H), 6.76 (d, *J* = 8.8 Hz, 1H), 4.90 (td, *J* = 6.6, 4.1 Hz, 1H), 4.62 (dd, *J* = 5.1, 3.1 Hz, 1H), 4.53 - 4.45 (m, 1H), 4.37 - 4.10 (m, 2H), 3.98 (qd, *J* = 10.0, 5.4 Hz, 2H), 1.39 (d, *J* = 6.5 Hz, 3H) ppm.[1]

**1'-(R,R):** $^1$H NMR (400 MHz, $CDCl_3$) δ 7.31 (d, *J* = 2.5 Hz, 1H), 7.11 (dd, *J* = 8.8, 2.5 Hz, 1H), 6.76 (d, *J* = 8.8 Hz, 1H), 4.90 (td, *J* = 6.6, 4.1 Hz, 1H), 4.69 - 4.57 (m, 1H), 4.57 - 4.46 (m, 1H), 4.36 - 4.09 (m, 2H), 3.98 (qd, *J* = 10.0, 5.4 Hz, 2H), 1.39 (d, *J* = 6.5 Hz, 3H) ppm.

**1'-(R,S):** $^1$H NMR (400 MHz, $CDCl_3$) δ 7.30 (d, *J* = 2.5 Hz, 1H), 7.11 (dd, *J* = 8.8, 2.5 Hz, 1H), 6.76 (d, *J* = 8.8 Hz, 1H), 4.88 (td, *J* = 6.4, 4.3 Hz, 1H), 4.65 - 4.57 (m, 1H), 4.50 (td, *J* = 3.9, 3.3, 1.5 Hz, 1H), 4.32 - 4.12 (m, 2H), 4.01 (dd, *J* = 10.0, 6.3 Hz, 1H), 3.93 (dd, *J* = 10.0, 4.3 Hz, 1H), 1.43 (s, 3H) ppm.

**S-10:** $^1$H NMR (400 MHz, $CDCl_3$) δ 7.44 (d, *J* = 1.4 Hz, 1H), 7.27 - 7.25 (m, 1H), 7.10 (d, *J* = 7.5 Hz, 1H), 4.87 (t, *J* = 2.9 Hz, 1H), 4.75 (t, *J* = 2.9 Hz, 1H), 4.42 (s, 2H), 3.97 (t, *J* = 2.9 Hz, 1H), 3.90 (t, *J* = 2.9 Hz, 1H), 1.51 (s, 6H) ppm.

**R-10:** $^1$H NMR (400 MHz, $CDCl_3$) δ 7.46 (d, *J* = 1.4 Hz, 1H), 7.31 - 7.27 (m, 1H), 7.18 (d, *J* = 7.5 Hz, 1H), 4.87 (t, *J* = 7.2 Hz, 1H), 4.75 (t, *J* = 7.3 Hz, 1H), 4.30 (s, 2H), 4.01 - 3.03 (m, 2H), 1.47 (s, 6H) ppm.

**S-10':** $^1$H NMR (400 MHz, $CDCl_3$) δ 7.54 (d, *J* = 1.4 Hz, 1H), 7.32 (dd, *J* = 7.5, 1.4 Hz, 1H), 7.17 (d, *J* = 7.5 Hz, 1H), 4.87 (t, *J* = 3.2 Hz, 1H), 4.75 (t, *J* = 3.2 Hz, 1H), 4.14 (s, 2H), 4.10 (t, *J* = 3.2 Hz, 1H), 4.04 (t, *J* = 3.2 Hz, 1H), 1.47 (s, 6H) ppm.

**R-10':** $^1$H NMR (400 MHz, $CDCl_3$) δ 7.58 (d, *J* = 1.4 Hz, 1H),7.34 - 7.30 (m, 1H), 7.19 (d, *J* = 7.5 Hz, 1H), 4.91 (t, *J* = 2.9 Hz, 1H), 4.78 (t, *J* = 2.9 Hz, 1H), 4.13 (s, 2H), 3.95 - 3.88 (m, 2H), 1.45 (s, 6H) ppm.

**19-(R,R):$^1$H** NMR (400 MHz, $CDCl_3$) δ 7.47-7.43 (m, 1H), 7.29 (dd, *J* = 7.5, 1.4 Hz, 1H), 7.16 (d, *J* = 7.5 Hz, 1H), 4.94-4.88 (m, 2H), 4.75 (t, *J* = 3.0 Hz, 1H), 4.40-4.36 (m, 1H), 3.96-3.93 (m, 1H) 3.86-3.82 (m, 2H),1.55-1.48 (m, 2H), 0.99 (t, *J* = 6.8 Hz, 3H) ppm.

**19-(R,S):** $^1$H NMR (400 MHz, $CDCl_3$) δ 7.41 (d, *J* = 1.4 Hz, 1H), 7.26-7.22 (m, 1H), 7.06 (d, *J* = 7.5 Hz, 1H), 5.12-5.08 (m, 1H), 4.87 (t, *J* = 2.8 Hz, 1H), 4.75 (t, *J* = 2.8 Hz, 1H), 4.24-4.20 (m, 1H), 4.06 - 3.90 (m, 2H), 3.86 (t, *J* = 2.8 Hz,

1H), 1.83 - 1.61 (m, 1H), 1.50-1.46 (m, 1H), 0.99 (t, *J* = 6.7 Hz, 3H) ppm.

**19-(S,R):** $^1$H NMR (400 MHz, $CDCl_3$) δ 7.41 (d, *J* = 1.4 Hz, 1H), 7.29-7.25 (m, 1H), 7.07 (d, *J* = 7.4 Hz, 1H), 5.11-5.05 (m, 1H), 4.87 (t, *J* = 2.9 Hz, 1H), 4.75 (t, *J* = 2.9 Hz, 1H), 4.56-4.48 (m, 1H), 4.00 - 3.69 (m, 3H), 1.55-1.47 (m, 2H), 0.98 (t, *J* = 6.7 Hz, 3H) ppm.

**19-(S,S) :** $^1$H NMR (400 MHz, $CDCl_3$) δ 7.46 (d, *J* = 1.4 Hz, 1H), 7.33-7.27 (m, 2H), 5.11-5.06 (m, 1H), 4.87 (t, *J* = 3.5 Hz, 1H), 4.75 (t, *J* = 3.5 Hz, 1H), 4.33-4.30 (m, 1H), 4.10 - 3.82 (m, 3H), 1.67 - 1.41 (m, 2H), 0.98 (t, *J* = 6.7 Hz, 3H) ppm.

**19'-(S,S):** $^1$H NMR (400 MHz, $CDCl_3$) δ 7.55 (d, *J* = 1.4 Hz, 1H), 7.35-7.32 (m, 1H), 7.16 (d, *J* = 7.5 Hz, 1H), 4.87 (t, *J* = 2.8 Hz, 1H), 4.75 (t, *J* = 2.9 Hz, 1H), 4.24 - 3.97 (m, 2H), 3.88 - 3.39 (m, 3H), 1.96 - 1.51 (m, 2H), 0.99 (t, *J* = 6.7 Hz, 3H) ppm.

**19'-(S,R):** $^1$H NMR (400 MHz, $CDCl_3$) δ 7.54 (d, *J* = 1.4 Hz, 1H), 7.32 (dd, *J* = 7.5, 1.4 Hz, 1H), 7.18 (d, *J* = 7.5 Hz, 1H), 4.87 (t, *J* = 2.9 Hz, 1H), 4.75 (t, *J* = 2.9 Hz, 1H), 4.27 - 4.04 (m, 2H), 4.03 - 3.79 (m, 3H), 1.91 - 1.58 (m, 2H), 0.98 (t, *J* = 6.7 Hz, 3H) ppm.

**19'-(R,R):** $^1$H NMR (400 MHz, $CDCl_3$) δ 7.55 (d, *J* = 1.5 Hz, 1H), 7.34-7.31 (m, 1H), 7.19 *(d, J* = 7.5 Hz, 1H), 4.87 (t, *J* = 3.1 Hz, 1H), 4.75 (t, *J* = 3.1 Hz, 1H), 4.31-4.27(m, 1H), 4.20 - 3.95 (m, 3H), 3.85-3.81 (m, 1H), 1.89 - 1.72 (m, 1H), 1.71 - 1.54 (m, 1H), 0.99 (t, *J* = 6.7 Hz, 3H) ppm.

**19'-(R,S):** $^1$H NMR (400 MHz, $CDCl_3$) δ 7.54 (d, *J* = 1.4 Hz, 1H), 7.32 (dd, *J* = 7.5, 1.4 Hz, 1H), 7.18-7.16 (m, 1H), 4.87 (t, *J* = 2.9 Hz, 1H), 4.75 (t, *J* = 2.9 Hz, 1H), 4.12 - 4.00 (m, 2H), 3.99 (t, *J* = 2.9 Hz, 1H), 3.92 (t, *J* = 2.8 Hz, 1H),3.86-3.83 (m, 1H), 1.73-1.71 (m, 2H), 0.97 (t, *J* = 6.7 Hz, 3H) ppm.

1.2.2 Determination of Specific Rotation of Compounds

The specific optical rotation was measured at 25°C using chloroform as solvent at a concentration of 1 mg/mL. The specific data are as follows:

1-(S,R): $[\alpha]^{25}$D = -10.00
1-(S,S): $[\alpha]^{25}$D = -9.00
1-(R,R): $[\alpha]^{25}$D =+10.00
1-(R,S): $[\alpha]^{25}$D =+10.00
1'-(R,S): $[\alpha]^{25}$D = -8.00
1'-(R,R): $[\alpha]^{25}$D = +41.00
1'-(S,R): $[\alpha]^{25}$D = +7.00
1'-(S,S): $[\alpha]^{25}$D = -38.00
S-10: $[\alpha]^{25}$D = -12.00
R-10: $[\alpha]^{25}$D = +11.00
S-10': $[\alpha]^{25}$D = -7.00
R-10': $[\alpha]^{25}$D = +6.00
19-(R,R): $[\alpha]^{25}$D = +37.00
19-(R,S): $[\alpha]^{25}$D = -10.00
19-(S,R): $[\alpha]^{25}$D = +11.00
19-(S,S): $[\alpha]^{25}$D = -38.00
19'-(R,R): $[\alpha]^{25}$D = +9.00
19'-(R,S): $[\alpha]^{25}$D = +11.00
19'-(S,R): $[\alpha]^{25}$D = -12.00
19'-(S,S): $[\alpha]^{25}$D = -8.00

According to the results of specific rotation, which are consistent with the absolute configuration speculated from the analysis of H NMR spectroscopy, therefore the configuration of the product should be correct.

**Example 6 Synthesis of Compound 1**

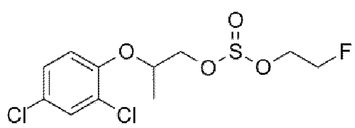

**1**

Step 1:

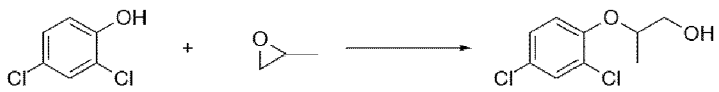

i-1 ii-1 iii-1

2,4-dichlorophenol (1 g, 6.2 mmol) was added into a reaction flask, and dissolved in 20 mL of DMF, then propylene oxide (722 mg, 7.6 mmol), and cesium carbonate (8 g, 24.8 mmol) were added, the reaction solution was heated in an oil bath at 100°C and reacted for 6 hours, then the reaction was monitored by TLC until it was completed, 100 mL of water was added and extracted with ethyl acetate (30 mL × 3), the organic phases were collected after washing with saturated saline solution, dried over anhydrous sodium sulfate, concentrated under reduced pressure, and purified by separation. Chiral preparation was performed using Instrument: Shimadzu high-pressure liquid phase chromatography, LC20AR; Chiral Column: CHIRALPAK® Positive Phase IG column, 4.6 mmI.D.×250 mm, diameter: 5 μm; Mobile Phase: n-hexane: isopropanol=90:10) to obtain compound iii-1(781 mg, colorless transparent liquid).

Step 2:

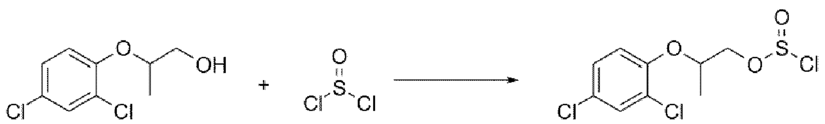

iii-1 iv-1 v-1

Thionyl chloride (293 mg, 2.5 mmol) was added into a reaction flask, and dissolved in 20 mL of dichloromethane, the reaction flask was transferred to an ice bath at 0°C and compounds iii-1 (356 mg, 1.6 mmol) was added dropwise slowly therein while stirring. After the dropwise addition was completed, the reaction flask was warmed to room temperature and reacted for 10 hours. The reaction was monitored by TLC until it was completed, and the reaction solution was concentrated under reduced pressure to obtain a light yellow oil, which was the crude product of compound v-1, and was ready for later use.

Step 3:

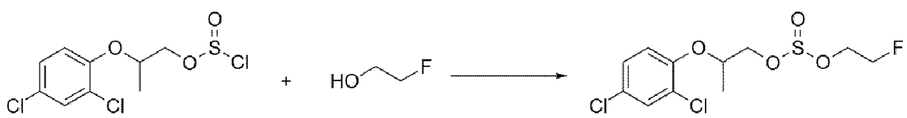

v-1 vi-1 **1**

Compound vi-1 (123 mg, 1.9 mmol) was added into a reaction flask, triethylamine (243 mg, 2.4 mmol) was added thereto, then the reaction flask was transferred to an ice bath at 0°C and compounds v-1 (480 mg, 1.6 mmol) was added dropwise slowly therein while stirring. After the dropwise addition was completed, the reaction flask was warmed to room temperature and reacted for 6 hours. The reaction was monitored by TLC until it was completed, then 100 mL of water was added to the reaction solution and extracted with dichloromethane (30 mL × 3), the organic phases were collected after washing with saturated saline solution, dried over anhydrous sodium sulfate, concentrated under reduced pressure, and purified by column chromatography to obtain compound 1 as an oil (423 mg).

$^1$H NMR (400 MHz, $CDCl_3$) δ 7.50 (d, *J* = 1.4 Hz, 1H), 7.30 - 7.27 (m, 1H), 7.08 (d, *J* = 7.5 Hz, 1H), 4.87 (t, *J* = 2.9 Hz, 1H), 4.75 (t, *J* = 2.9 Hz, 1H), 4.64 - 4.54 (m, 1H), 4.51 - 4.46 (m, 1H), 4.14- 4.10 (m, 1H), 3.95 (t, *J* = 2.8 Hz, 1H), 3.88 (t, *J* = 2.9

Hz, 1H), 1.40 (d, *J* = 5.7 Hz, 3H) ppm.
HRMS (ESI) Calcd. For $C_{11}H_{14}Cl_2FO_4SNa^+$ $[M+Na]^+$ 352.9768; Found: 352.9789, 354.9773

**Example 7 Synthesis of Compound 1'**

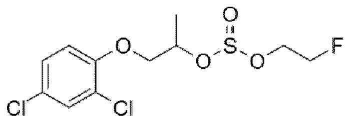

**1'**

Step 1:

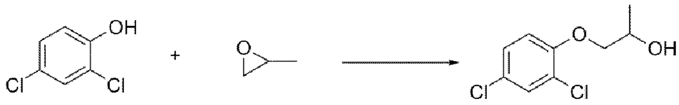

i-1 ii-1 iii-1'

The target compound iii-1' was obtained according to the synthesis in step 1 of Example 6 through chiral preparation.

Step 2: It was same as step 2 in Example 1.

Step 3: It was same as step 3 in Example 1.

The target compound 1' (674 mg, colorless transparent liquid) was synthesized.

$^1H$ NMR (400 MHz, $CDCl_3$) δ 7.39 (d, *J* = 2.5 Hz, 1H), 7.20 (dd, *J* = 8.8, 2.5 Hz, 1H), 6.85 (d, *J* = 8.8 Hz, 1H), 5.01-4.93 (m, 1H), 4.74 - 4.66 (m, 1H), 4.63 - 4.55 (m, 1H), 4.40-4.31 (m, 1H), 4.31-4.22 (m, 1H), 4.10 (dd, *J* = 10.0, 6.3 Hz, 1H), 4.02 (dd, *J* = 10.0, 4.3 Hz, 1H), 1.52 (d, *J* = 6.5 Hz, 3H) ppm.

HRMS (ESI) Calcd. For $C_{11}H_{14}Cl_2FO_4SNa^+$ $[M+Na]^+$ 352.9768; Found: 352.9786, 354.9758 .

**Example 8 Synthesis of Compound 10**

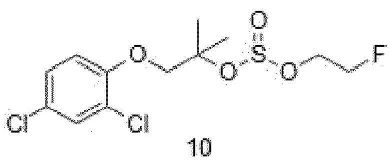

10

Step 1:

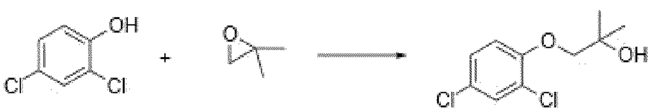

i-1 ii-10 iii-10

The target compound iii-10 was obtained according to the synthesis in step 1 of Example 6 through chiral preparation.

Step 2 was the same as step 2 in Example 1.

Step 3 was the same as step 3 in Example 1.

The target compound 10 (685 mg, colorless transparent liquid) was synthesized.

$^1H$ NMR (400 MHz, $CDCl_3$) δ 7.46 (d, *J* = 1.4 Hz, 1H), 7.29 (dd, *J* = 7.5, 1.4 Hz, 1H), 7.18 (d, *J* = 7.5 Hz, 1H), 4.87 (t, *J* = 7.2 Hz, 1H), 4.75 (t, *J* = 7.3 Hz, 1H), 4.30 (s, 2H), 4.03 - 3.03 (m, 2H), 1.47 (s, 6H) ppm.

HRMS (ESI) Calcd. For $C_{12}H_{16}Cl_2FO_4S^+$ $[M+H]^+$ 345.0125; Found:345.0143.

**Example 9 Synthesis of Compound 10'**

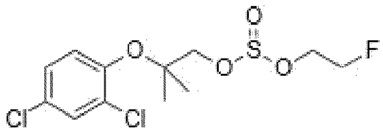

10'

Step 1:

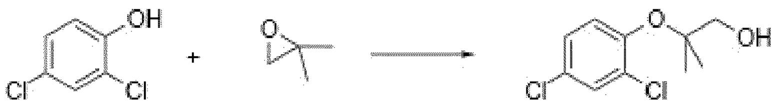

i-1 ii-10 iii-10'

The target compound iii-10' was obtained according to the synthesis in step 1 of Example 6 through chiral preparation.

Step 2 was the same as step 2 in Example 1.

Step 3 was the same as step 3 in Example 1.

The target compound 10' (311 mg, colorless transparent liquid) was synthesized.

$^1H$ NMR (400 MHz, $CDCl_3$) δ 7.62 (d, *J* = 1.4 Hz, 1H), 7.29 - 7.27 (m, 1H), 7.16 (d, *J* = 7.5 Hz, 1H), 4.87 (t, *J* = 2.9 Hz, 1H), 4.75 (t, *J* = 2.9 Hz, 1H), 4.13 (s, 2H), 4.04 (t, *J* = 2.9 Hz, 1H), 3.97 (t, *J* = 2.9 Hz, 1H), 1.37 (s, 6H).

HRMS (ESI) Calcd. For $C_{12}H_{16}Cl_2FO_4S^+$ $[M+H]^+$ 345.0125; Found:345.0126.

**Example 10 Synthesis of Compound 19**

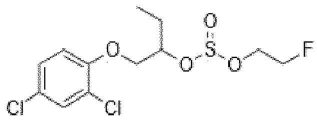

19

Step 1:

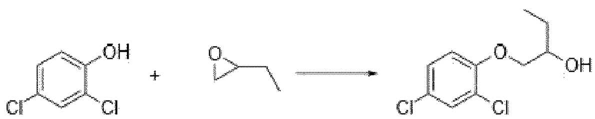

i-1 ii-19 iii-19

The target compound iii-19 was obtained according to the synthesis in step 1 of Example 6 through chiral preparation.

Step 2 was the same as step 2 in Example 1.

Step 3 was the same as step 3 in Example 1.
The target compound 19 (692 mg, colorless transparent liquid) was synthesized.
$^1$H NMR (400 MHz, $CDCl_3$) $\delta$ 7.45 (d, $J$ = 1.6 Hz, 1H), 7.31-7.27 (m, 1H), 7.16 (d, $J$ = 7.5 Hz, 1H), 4.97-4.84 (m, 2H), 4.75 (t, $J$ = 3.0 Hz, 1H), 4.39-4.34 (m, 1H), 3.99-3.77 (m, 3H), 1.56-1.43 (m, 2H), 0.99 (t, $J$ = 6.8 Hz, 3H) ppm.
HRMS (ESI) Calcd. For $C_{12}H_{15}O_4Cl_2FS^+$ $[M+H]^+$ 344.0052; Found:344.0026.

**Example 11 Synthesis of Compound 19'**

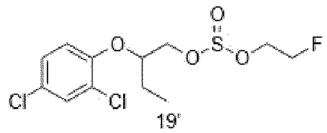

19'

Step 1:

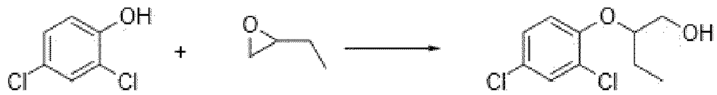

i-1 ii-19 iii-19'

The target compound iii-19' was obtained according to the synthesis in step 1 of Example 6 through chiral preparation.
Step 2 was the same as step 2 in Example 1.
Step 3 was the same as step 3 in Example 1.
The target compound 19' (309 mg, colorless transparent liquid) was synthesized.
$^1$H NMR (400 MHz, $CDCl_3$) $\delta$ 7.55 (d, $J$ = 1.4 Hz, 1H), 7.32 (dd, $J$ = 7.5, 1.4 Hz, 1H), 7.21-7.17 (m, 1H), 4.87 (t, $J$ = 3.1 Hz, 1H), 4.75 (t, $J$ = 3.1 Hz, 1H),4.61-4.57 (m, 1H), 4.25 - 3.88 (m, 4H), 1.80-1.75 (m, 1H)), 1.71 - 1.54 (m, 1H), 0.96 (t, $J$ = 6.7 Hz, 3H) ppm.
HRMS (ESI) Calcd. For $C_{12}H_{15}O_4Cl_2FS^+$ $[M+H]^+$ 344.0052; Found:344.0071.

**Example 12 Synthesis of Compound 22**

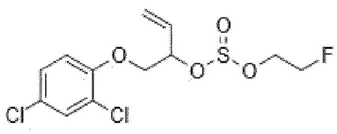

22

Step 1:

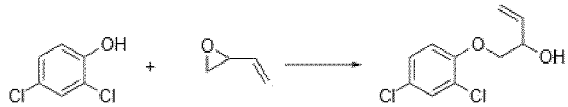

i-1 ii-22 iii-22

The target compound iii-22 was obtained according to the synthesis in step 1 of Example 6 through chiral preparation.

Step 2 was the same as step 2 in Example 1.

Step 3 was the same as step 3 in Example 1.

The target compound 22 (671 mg, colorless transparent liquid) was synthesized.

$^1$H NMR (400 MHz, $CDCl_3$) δ 7.44 (d, *J* = 1.4 Hz, 1H), 7.30 (dd, *J* = 7.5, 1.6 Hz, 1H), 7.20 (d, *J* = 7.5 Hz, 1H), 6.04 - 5.93 (m, 1H), 5.51 - 5.34 (m, 1H), 5.25 - 5.12 (m, 2H), 4.87 (t, *J* = 3.0 Hz, 1H), 4.75 (t, *J* = 3.0 Hz, 1H), 4.39 (dd, *J* = 12.5, 2.4 Hz, 1H), 4.13 - 4.02 (m, 2H), 3.99 (t, *J*= 3.0 Hz, 1H) ppm.

HRMS (ESI) Calcd. For $C_{12}H_{13}O_4Cl_2FS^+$ $[M+H]^+$ 341.9896; Found:341.9857.

**Example 13 Synthesis of Compound 22'**

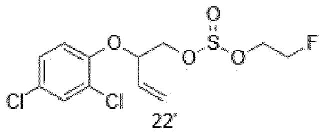

22'

Step 1:

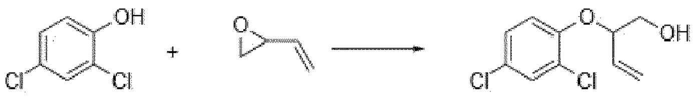

i-1 ii-22 iii-22'

The target compound iii-22' was obtained according to the synthesis in step 1 of Example 6 through chiral preparation.

Step 2 was the same as step 2 in Example 1.

Step 3 was the same as step 3 in Example 1.

The target compound 22' (297 mg, colorless transparent liquid) was synthesized.

$^1$H NMR (400 MHz, $CDCl_3$) δ 7.45 (d, *J* = 1.4 Hz, 1H), 7.25 (dd, *J* = 7.5, 1.4 Hz, 1H), 7.18 (d, *J* = 7.5 Hz, 1H), 5.95 - 5.80 (m, 1H), 5.31 - 5.15 (m, 2H), 4.87 (t, *J* = 3.0 Hz, 1H), 4.75 (t, *J* = 3.0 Hz, 1H), 4.54 - 4.50 (m, 1H), 4.14 (dd, *J* = 12.5, 5.1 Hz, 1H), 3.75 (dd, *J*= 12.4, 5.0 Hz, 1H), 3.65 (t, *J* = 3.0 Hz, 1H), 3.59 (t, *J* = 3.0 Hz, 1H) ppm.

HRMS (ESI) Calcd. For $C_{12}H_{13}O_4Cl_2FS^+$ $[M+H]^+$ 341.9896; Found:341.9903.

**Example 14 Synthesis of Compound 25**

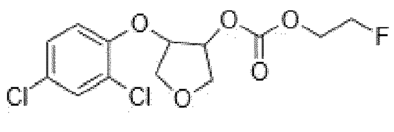

25

Step 1:

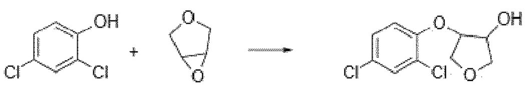

i ii-25 iii-25

According to the synthetic method in step 1 of Example 6, the propylene oxide was replaced with 3,4-epoxytetrahydrofuran to obtain the target compound iii-25.

Step 2 was the same as step 2 in Example 1.

Step 3 was the same as step 3 in Example 1.

The target compound 25 (703 mg, colorless transparent liquid) was synthesized.

**Example 15 Synthesis of Compound 28**

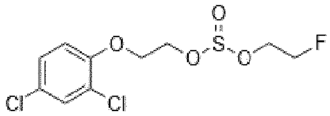

28

Step 1:

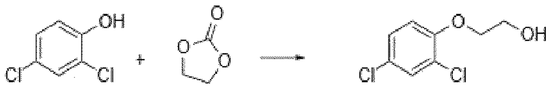

i ii-28 iii-28

According to the synthetic method in step 1 of Example 6, the propylene oxide was replaced with ethylene carbonate to obtain the target compound iii-28.

Step 2 was the same as step 2 in Example 1.

Step 3 was the same as step 3 in Example 1.

The target compound 22 (671 mg, colorless transparent liquid) was synthesized..

The remaining compounds were synthesized according to the above synthesis methods, and structures of all compounds are shown in Table 1:

Table 1

| Nos. | $R_1$ | $R_2$ | $R_3$ | R3' | $R_4$ | R4' | $R_5$ |
|---|---|---|---|---|---|---|---|
| 1 | 2-Cl | 4-Cl | $CH_3$ | H | H | H | $CH_2CH_2F$ |
| 1' | 2-Cl | 4-Cl | H | H | $CH_3$ | H | $CH_2CH_2F$ |
| 2 | 2-Cl | 4-Cl | $CH_3$ | H | H | H | $CH_2CH_2Cl$ |
| 2' | 2-Cl | 4-Cl | H | H | $CH_3$ | H | $CH_2CH_2Cl$ |
| 3 | 2-Cl | 4-Cl | $CH_3$ | H | H | H | $CH_2CH_2Br$ |
| 3' | 2-Cl | 4-Cl | H | H | $CH_3$ | H | $CH_2CH_2Br$ |
| 4 | 3-Cl | 5-Cl | $CH_3$ | H | H | H | $CH_2CH_2F$ |
| 4' | 3-Cl | 5-Cl | H | H | $CH_3$ | H | $CH_2CH_2F$ |
| 5 | 3-Cl | 5-Cl | $CH_3$ | H | H | H | $CH_2CH_2Cl$ |
| 5' | 3-Cl | 5-Cl | H | H | $CH_3$ | H | $CH_2CH_2Cl$ |
| 6 | 3-Cl | 5-Cl | $CH_3$ | H | H | H | $CH_2CH_2Br$ |
| 6' | 3-Cl | 5-Cl | H | H | $CH_3$ | H | $CH_2CH_2Br$ |
| 7 | 2-Cl | 5-Cl | $CH_3$ | H | H | H | $CH_2CH_2F$ |
| 7' | 2-Cl | 5-Cl | H | H | $CH_3$ | H | $CH_2CH_2F$ |
| 8 | 2-Cl | 5-Cl | $CH_3$ | H | H | H | $CH_2CH_2Cl$ |
| 8' | 2-Cl | 5-Cl | H | H | $CH_3$ | H | $CH_2CH_2Cl$ |

(continued)

| Nos. | $R_1$ | $R_2$ | $R_3$ | R3' | $R_4$ | R4' | $R_5$ |
|---|---|---|---|---|---|---|---|
| 9 | 2-Cl | 5-Cl | $CH_3$ | H | H | H | $CH_2CH_2Br$ |
| 9' | 2-Cl | 5-Cl | H | H | $CH_3$ | H | $CH_2CH_2Br$ |
| 10 | 2-Cl | 4-Cl | H | H | $CH_3$ | $CH_3$ | $CH_2CH_2F$ |
| 10' | 2-Cl | 4-Cl | $CH_3$ | $CH_3$ | H | H | $CH_2CH_2F$ |
| 11 | 2-Cl | 4-Cl | H | H | $CH_3$ | $CH_3$ | $CH_2CH_2Cl$ |
| 11' | 2-Cl | 4-Cl | $CH_3$ | $CH_3$ | H | H | $CH_2CH_2Cl$ |
| 12 | 2-Cl | 4-Cl | H | H | $CH_3$ | $CH_3$ | $CH_2CH_2Br$ |
| 12' | 2-Cl | 4-Cl | $CH_3$ | $CH_3$ | H | H | $CH_2CH_2Br$ |
| 13 | 3-Cl | 5-Cl | H | H | $CH_3$ | $CH_3$ | $CH_2CH_2Br$ |
| 13' | 3-Cl | 5-Cl | $CH_3$ | $CH_3$ | H | H | $CH_2CH_2F$ |
| 14 | 3-Cl | 5-Cl | H | H | $CH_3$ | $CH_3$ | $CH_2CH_2F$ |
| 14' | 3-Cl | 5-Cl | $CH_3$ | $CH_3$ | H | H | $CH_2CH_2Cl$ |
| 15 | 3-Cl | 5-Cl | H | H | $CH_3$ | $CH_3$ | $CH_2CH_2Cl$ |
| 15' | 3-Cl | 5-Cl | $CH_3$ | $CH_3$ | H | H | $CH_2CH_2Br$ |
| 16 | 2-Cl | 5-Cl | H | H | $CH_3$ | $CH_3$ | $CH_2CH_2Br$ |
| 16' | 2-Cl | 5-Cl | $CH_3$ | $CH_3$ | H | H | $CH_2CH_2F$ |
| 17 | 2-Cl | 5-Cl | H | H | $CH_3$ | $CH_3$ | $CH_2CH_2F$ |
| 17' | 2-Cl | 5-Cl | $CH_3$ | $CH_3$ | H | H | $CH_2CH_2Cl$ |
| 18 | 2-Cl | 5-Cl | H | H | $CH_3$ | $CH_3$ | $CH_2CH_2Cl$ |
| 18' | 2-Cl | 5-Cl | $CH_3$ | $CH_3$ | H | H | $CH_2CH_2Br$ |
| 19 | 2-Cl | 4-Cl | H | H | $CH_2CH_3$ | H | $CH_2CH_2F$ |
| 19' | 2-Cl | 4-Cl | $CH_2CH_3$ | H | H | H | $CH_2CH_2F$ |
| 20 | 3-Cl | 5-Cl | H | H | $CH_2CH_3$ | H | $CH_2CH_2F$ |
| 20' | 3-Cl | 5-Cl | $CH_2CH_3$ | H | H | H | $CH_2CH_2F$ |
| 21 | 2-Cl | 5-Cl | H | H | $CH_2CH_3$ | H | $CH_2CH_2F$ |
| 21' | 2-Cl | 5-Cl | $CH_2CH_3$ | H | H | H | $CH_2CH_2F$ |
| 22 | 2-Cl | 4-Cl | H | H | $CH=CH_2$ | H | $CH_2CH_2F$ |
| 22' | 2-Cl | 4-Cl | $CH=CH_2$ | H | H | H | $CH_2CH_2F$ |
| 23 | 3-Cl | 5-Cl | H | H | $CH=CH_2$ | H | $CH_2CH_2F$ |
| 23' | 3-Cl | 5-Cl | $CH=CH_2$ | H | H | H | $CH_2CH_2F$ |
| 24 | 2-Cl | 5-Cl | H | H | $CH=CH_2$ | H | $CH_2CH_2F$ |
| 24' | 2-Cl | 5-Cl | $CH=CH_2$ | H | H | H | $CH_2CH_2F$ |
| 25 | 2-Cl | 4-Cl | | | | | $CH_2CH_2F$ |
| 26 | 3-Cl | 5-Cl | | | | | $CH_2CH_2F$ |
| 27 | 2-Cl | 5-Cl | | | | | $CH_2CH_2F$ |
| 28 | 2-Cl | 4-Cl | H | H | H | H | $CH_2CH_2F$ |
| 29 | 3-Cl | 5-Cl | H | H | H | H | $CH_2CH_2F$ |
| 30 | 2-Cl | 5-Cl | H | H | H | H | $CH_2CH_2F$ |

**Test Example 1 Effect on Hatching of Pest Eggs of *Tetranychus Cinnabarinus* by Compounds 1-(S,S), 1-(S,R), 1-(R,R), 1-(R,S), 1'-(S,S), 1'-(S,R), 1'-(R,R), 1'-(R,S), Compound 1, Compound 1', Compounds S-10, R-10, S-10', R-10', Compound 10, Compound 10', Compounds 19-(S,S), 19-(S,R), 19-(R,R), 19-(R,S), 19'-(S,S), 19'-(S,R), 19'-(R,R), 19'-(R,S), Compound 19, and Compound 19'**

1. Experimental Method:

(1) Preparation of leaf discs containing eggs: 20 female adult mites of *Tetranychus cinnabarinus* were transferred onto leaf discs of Vicia faba with a diameter of 2.0 cm (with wetted filter paper at the bottom), then covered it with a lidded culture dish and cultivated under moisturizing conditions, the adult mites were removed within 36 hours and microscopic examination and counting were performed on leaf discs containing eggs.
(2) Investigation of egg population base: Before pesticide soaking, a microscopic examination was conducted on the egg basic number of each leaf disc, with 2 replicates set for each treatment.
(3) Pesticide soaking treatment: The *leaf butterflies* loaded with mite eggs were soaked in clear water and sulfite compounds for 10 seconds, respectively, then taken out and cultivated under moisturizing conditions. Each treatment should be repeated for no less than 3 times.
(4) Cultivation and observation: The treated mite eggs and *leaf butterflies* were cultivated under normal conditions. Five days after insecticide delivery, the hatching status of mite eggs was investigated.
Note: The temperature and humidity conditions of the constant temperature and humidity incubator after the treatment should be controlled so as to avoid excessive temperature differences, which may cause generation and dripping of condensate water in the culture dish, resulting in abnormal death of eggs due to soaking in water; it is also necessary to ensure sufficient strong ambient light, without directing irradiating the leaves.
(5) Survey of Results: The experimental materials of each treatment group were regularly hydrated and moisturized, and the hatching of eggs was observed. On the 7th day after insecticide delivery, the number of hatched eggs for each treatment was recorded, and the survey of results was recorded in the original notebook. According to the experimental requirements and characteristics of agents, the survey time can be shortened or extended.

Survey indicators:

1) Surveying and recording the number of hatched eggs for each treatment.
2) Photographing and recording whether there was any pesticide damage to the leaf discs of Vicia faba.
3) Recording the developmental status of experimental mite eggs, behavior status of nymph mites, including abnormal phenomena such as delayed or stopped development of mite eggs, difficulty in breaking hull for nymph mites, or painful struggles after hatching, etc.
(6) Calculation method: Based on the survey data, the prevention and control effect of each treatment was calculated according to the following formula, and the calculation results were kept to two decimal places.

Egg hatching rate (%)=number of hatched eggs/total number of treated eggs*100

Prevention and control effect (%)=(hatching rate of eggs in control area - hatching rate of eggs in treatment area)/ hatching rate of eggs in control area)* 100

The experimental design is as shown in Table 2.

Table 2 Experimental Design

| Treatment numbers | Experimental treatments | Experimental concentrations (mg/L) | Application methods | Application timings |
|---|---|---|---|---|
| T1 | Compound 1 | 1 | | |
| T2 | Compound 1 | 0.5 | | |
| T3 | Compound 1 | 0.25 | | |

(continued)

| Treatment numbers | Experimental treatments | Experimental concentrations (mg/L) | Application methods | Application timings |
|---|---|---|---|---|
| T4 | 1-(S,S) | 1 | Leaf disc | / |
| T5 | 1-(S,S) | 0.5 | | |
| T6 | 1-(S,S) | 0.25 | | |
| T7 | 1-(S,R) | 1 | | |
| T8 | 1-(S,R) | 0.5 | | |
| T9 | 1-(S,R) | 0.25 | | |
| T10 | 1-(R,R) | 1 | | |
| T11 | 1-(R,R) | 0.5 | | |
| T12 | 1-(R,R) | 0.25 | | |
| T13 | 1-(R,S) | 1 | | |
| T14 | 1-(R,S) | 0.5 | | |
| T15 | 1-(R,S) | 0.25 | | |
| T16 | Compound 1’ | 1 | | |
| T17 | Compound 1’ | 0.5 | | |
| T18 | Compound 1’ | 0.25 | | |
| T19 | 1’-(S,S) | 1 | | |
| T20 | 1’-(S,S) | 0.5 | | |
| T21<br>T22 | 1’-(S,S)<br>1’-(S,R) | 0.25<br>1 | | |
| T23 | 1’-(S,R) | 0.5 | | |
| T24 | 1’-(S,R) | 0.25 | | |
| T25 | 1’-(R,R) | 1 | | |
| T26 | 1’-(R,R) | 0.5 | | |
| T27 | 1’-(R,R) | 0.25 | | |
| T28 | 1’-(R, S) | 1 | | |
| T29 | 1’-(R,S) | 0.5 | | |
| T30 | 1’-(R, S) | 0.25 | | |
| T31 | Compound 10’ | 1 | | |
| T32 | Compound 10’ | 0.5 | | |
| T33 | Compound 10’ | 0.25 | | |
| T34 | S-10 | 1 | | |
| T35 | S-10 | 0.5 | | |
| T36 | S-10 | 0.25 | | |
| T37 | R-10 | 1 | | |
| T38 | R-10 | 0.5 | | |
| T39 | R-10 | 0.25 | | |
| T40 | Compound 10’ | 1 | | |
| T41 | Compound 10’ | 0.5 | | |
| T42 | Compound 10’ | 0.25 | | |

(continued)

| Treatment numbers | Experimental treatments | Experimental concentrations (mg/L) | Application methods | Application timings |
|---|---|---|---|---|
| T43 | S-10’ | 1 | | |
| T44 | S-10’ | 0.5 | | |
| T45 | S-10’ | 0.25 | | |
| T46 | R-10’ | 1 | | |
| T47 | R-10’ | 0.5 | | |
| T48 | R-10’ | 0.25 | | |
| T49 | Compound 19 | 1 | | |
| T50 | Compound 19 | 0.5 | | |
| T51 | Compound 19 | 0.25 | | |
| T52<br>T53 | 19-(S,S)<br>19-(S,S) | 1<br>0.5 | | |
| T54 | 19-(S,S) | 0.25 | | |
| T55 | 19-(S,R) | 1 | | |
| T56 | 19-(S,R) | 0.5 | | |
| T57 | 19-(S,R) | 0.25 | | |
| T58 | 19-(R,R) | 1 | | |
| T59 | 19-(R,R) | 0.5 | | |
| T60 | 19-(R,R) | 0.25 | | |
| T61 | 19-(R, S) | 1 | | |
| T62 | 19-(R, S) | 0.5 | | |
| T63 | 19-(R, S) | 0.25 | | |
| T64 | Compound 19’ | 1 | | |
| T65 | Compound 19’ | 0.5 | | |
| T66 | Compound 19’ | 0.25 | | |
| T67 | 19’-(S,S) | 1 | | |
| T68 | 19’-(S,S) | 0.5 | | |
| T69 | 19’-(S,S) | 0.25 | | |
| T70 | 19’-(S,R) | 1 | | |
| T71 | 19’-(S,R) | 0.5 | | |
| T72 | 19’-(S,R) | 0.25 | | |
| T73 | 19’-(R,R) | 1 | | |
| T74 | 19’-(R,R) | 0.5 | | |
| T75 | 19’-(R,R) | 0.25 | | |
| T76 | 19’-(R,S) | 1 | | |
| T77 | 19’-(R,S) | 0.5 | | |
| T78 | 19’-(R,S) | 0.25 | | |
| T79 | Solvent control | / | | |

(continued)

| Treatment numbers | Experimental treatments | Experimental concentrations (mg/L) | Application methods | Application timings |
|---|---|---|---|---|
| T80 | Clear water | / | | |
| 2. Experimental Results | | | | |

The results are shown in Table 3, compounds **1-(S,S), 1-(R,R), 1'-(S, R), 1'-(R,S),** compound **1,** compound **1', S-10, S-10', compound 10, compound 10', 19-(S, R), 19-(R,S), 19'-(S,S), 19'-(R,R),** compound **19, and** compound **19'** all showed excellent inhibitory effects on the hatching of pest eggs of *Tetranychus cinnabarinus* at 1 ppm.

Table 3

| Treatment | Basic number of eggs before insecticide delivery | | Number of hatched larvae 7 days after insecticide delivery | | Hatching rate (%) | |
|---|---|---|---|---|---|---|
| | Replicate 1 | Replicate 2 | Replicate 1 | Replicate 2 | Replicate 1 | Replicate 2 |
| T1 | 53 | 61 | 53 | 60 | 0.00 | 1.64 |
| T2 | 62 | 47 | 42 | 29 | 32.58 | 38.30 |
| T3 | 72 | 53 | 20 | 17 | 72.22 | 67.92 |
| T4 | 75 | 68 | 75 | 68 | 0.00 | 0.00 |
| T5 | 70 | 72 | 70 | 65 | 0.00 | 9.72 |
| T6 | 57 | 59 | 28 | 36 | 50.88 | 38.98 |
| T7 | 78 | 64 | 76 | 64 | 2.56 | 0.00 |
| T8 | 51 | 64 | 33 | 44 | 35.29 | 31.25 |
| T9 | 47 | 46 | 19 | 11 | 59.57 | 76.09 |
| T10 | 56 | 62 | 56 | 62 | 0.00 | 0.00 |
| T11 | 47 | 72 | 45 | 68 | 4.26 | 5.56 |
| T12 | 66 | 51 | 36 | 28 | 45.45 | 45.10 |
| T13 | 64 | 45 | 61 | 42 | 4.69 | 6.67 |
| T14 | 71 | 49 | 40 | 25 | 43.66 | 48.98 |
| T15 | 51 | 68 | 13 | 14 | 74.51 | 76.47 |
| T16 | 47 | 59 | 47 | 58 | 0.00 | 1.69 |
| T17 | 61 | 45 | 42 | 31 | 36.24 | 31.11 |
| T18 | 53 | 72 | 16 | 21 | 69.81 | 70.83 |
| T19 | 55 | 41 | 46 | 35 | 16.36 | 14.63 |
| T20 | 58 | 75 | 42 | 66 | 27.59 | 12.00 |
| T21 | 48 | 46 | 17 | 14 | 64.58 | 69.57 |
| T22 | 69 | 78 | 69 | 78 | 0.00 | 0.00 |
| T23 | 45 | 62 | 41 | 60 | 8.89 | 3.23 |
| T24 | 49 | 58 | 28 | 32 | 42.86 | 44.83 |
| T25 | 67 | 75 | 50 | 69 | 25.37 | 8.00 |
| T26 | 57 | 63 | 36 | 40 | 36.84 | 36.51 |
| T27 | 46 | 45 | 14 | 8 | 69.57 | 82.22 |
| T28 | 62 | 42 | 62 | 42 | 0.00 | 0.00 |
| T29 | 80 | 80 | 79 | 76 | 1.25 | 5.00 |

(continued)

| Treatment | Basic number of eggs before insecticide delivery | | Number of hatched larvae 7 days after insecticide delivery | | Hatching rate (%) | |
|---|---|---|---|---|---|---|
| | Replicate 1 | Replicate 2 | Replicate 1 | Replicate 2 | Replicate 1 | Replicate 2 |
| T30 | 48 | 96 | 22 | 68 | 54.17 | 29.17 |
| T31 | 81 | 78 | 80 | 78 | 1.23 | 0.00 |
| T32 | 69 | 63 | 47 | 41 | 31.88 | 34.92 |
| T33 | 73 | 56 | 24 | 20 | 67.12 | 64.29 |
| T34 | 78 | 84 | 78 | 84 | 0.00 | 0.00 |
| T35 | 65 | 59 | 62 | 57 | 4.62 | 3.39 |
| T36 | 81 | 67 | 49 | 39 | 39.51 | 41.79 |
| T37 | 74 | 64 | 68 | 60 | 8.11 | 6.25 |
| T38 | 48 | 62 | 28 | 36 | 42.67 | 41.94 |
| T39 | 56 | 77 | 15 | 20 | 73.21 | 74.03 |
| T40 | 76 | 68 | 75 | 66 | 1.32 | 2.94 |
| T41 | 68 | 83 | 47 | 58 | 30.88 | 30.12 |
| T42 | 82 | 57 | 27 | 17 | 67.07 | 70.18 |
| T43 | 59 | 84 | 59 | 84 | 0.00 | 0.00 |
| T44 | 88 | 49 | 81 | 46 | 7.95 | 6.12 |
| T45 | 76 | 65 | 44 | 37 | 42.11 | 43.77 |
| T46 | 59 | 67 | 54 | 62 | 8.47 | 7.46 |
| T47 | 63 | 71 | 36 | 39 | 42.86 | 45.07 |
| T48 | 81 | 56 | 21 | 13 | 74.07 | 76.79 |
| T49 | 71 | 65 | 71 | 65 | 0.00 | 0.00 |
| T50 | 49 | 82 | 32 | 57 | 34.69 | 30.49 |
| T51 | 54 | 76 | 15 | 23 | 72.22 | 69.74 |
| T52 | 59 | 63 | 50 | 54 | 15.25 | 14.29 |
| T53 | 67 | 61 | 53 | 48 | 20.90 | 21.31 |
| T54 | 58 | 73 | 20 | 22 | 65.52 | 69.86 |
| T55 | 60 | 68 | 60 | 68 | 0.00 | 0.00 |
| T56 | 78 | 57 | 74 | 54 | 5.13 | 5.26 |
| T57 | 62 | 57 | 35 | 31 | 43.55 | 45.61 |
| T58 | 47 | 66 | 40 | 55 | 14.89 | 16.67 |
| T59 | 69 | 50 | 44 | 34 | 36.23 | 36.00 |
| T60 | 72 | 75 | 18 | 18 | 75.00 | 76.00 |
| T61 | 59 | 67 | 59 | 67 | 0.00 | 0.00 |
| T62 | 48 | 56 | 47 | 54 | 2.08 | 3.57 |
| T63 | 63 | 58 | 38 | 34 | 39.68 | 41.38 |
| T64 | 53 | 63 | 52 | 63 | 1.89 | 0.00 |
| T65 | 72 | 61 | 47 | 40 | 34.72 | 34.43 |
| T66 | 47 | 71 | 14 | 19 | 70.21 | 73.24 |
| T67 | 56 | 49 | 56 | 49 | 0.00 | 0.00 |

(continued)

| Treatment | Basic number of eggs before insecticide delivery | | Number of hatched larvae 7 days after insecticide delivery | | Hatching rate (%) | |
|---|---|---|---|---|---|---|
| | Replicate 1 | Replicate 2 | Replicate 1 | Replicate 2 | Replicate 1 | Replicate 2 |
| T68 | 70 | 56 | 66 | 54 | 5.71 | 3.57 |
| T69 | 49 | 73 | 29 | 44 | 40.82 | 39.73 |
| T70 | 68 | 54 | 68 | 53 | 0.00 | 1.85 |
| T71 | 57 | 61 | 37 | 41 | 35.09 | 32.79 |
| T72 | 73 | 65 | 23 | 20 | 68.49 | 69.23 |
| T73 | 57 | 69 | 57 | 69 | 0.00 | 0.00 |
| T74 | 52 | 67 | 49 | 64 | 5.77 | 4.48 |
| T75 | 71 | 49 | 39 | 27 | 45.07 | 44.90 |
| T76 | 50 | 62 | 47 | 58 | 6.00 | 6.45 |
| T77 | 71 | 69 | 38 | 38 | 46.48 | 44.93 |
| T78 | 64 | 70 | 19 | 20 | 70.31 | 71.43 |
| T79 | 73 | 55 | 3 | 2 | 95.89 | 96.36 |
| T80 | 81 | 82 | 1 | 2 | 98.16 | |

**Test Example 2 Effect on Hatching of Eggs of *Thrips* by Compounds 1-(S,S), 1-(S,R), 1-(R,R), 1-(R,S), 1'-(S,S), 1'-(S,R), 1'-(R,R), 1'-(R,S), Compound 1, Compound 1', Compounds S-10, R-10, S-10', R-10', Compound 10, Compound 10', Compounds 19-(S,S), 19-(S,R), 19-(R,R), 19-(R,S), 19'-(S,S), 19'-(S,R), 19'-(R,R), 19'-(R,S), Compound 19, and Compound 19'**

This test adopted the method in Test Example 1 of technical section, mainly used leaf disc method to explore the inhibitory effect on hatching of eggs of *Thrips* by **1-(S,S), 1-(S,R), 1-(R,R), 1-(R,S), 1'-(S,S), 1'-(S,R), 1'-(R,R), 1'-(R,S), compound 1, compound 1' , S-10, R-10, S-10', R-10', compound 10, compound 10', compound 19-(S,S) , 19-(S,R), 19-(R,R), 19-(R,S), 19'-(S,S) ,19'-(S,R), 19'-(R,R), 19'-(R,S), compound 19, and compound 19'.** The concentration of all compounds was 100 ppm.

The experiments results are shown in Table 4, compounds **1-(S,S), 1-(R,R), 1'-(S, R), 1'-(R,S), compound 1, compound 1', S-10, S-10', compound 10, compound 10', 19-(S, R), 19-(R,S), 19'-(S,S), 19'-(R,R),** compound **19, and** compound **19'** all showed strong inhibitory activities on the hatching of eggs of *Thrips* at 100 ppm.

Table 4

| Treatme nt | Basic number of eggs before insecticide delivery | | | Number of hatched larvae 5 days after insecticide delivery | | | Hatching rate (%) | | |
|---|---|---|---|---|---|---|---|---|---|
| | Replic ate 1 | Replic ate 2 | Replic ate 3 | Replicat e 1 | Replicat e2 | Replicate 3 | Repli cate 1 | Repli cate 2 | Repli cate 3 |
| Compou nd **1** | 77 | 127 | 92 | 0 | 2 | 0 | 0.00 | 1.57 | 0.00 |
| **1-(S,S)** | 83 | 105 | 138 | 0 | 0 | 0 | 0.00 | 0.00 | 0.00 |
| **1-(S,R)** | 141 | 96 | 107 | 17 | 8 | 11 | 12.06 | 8.33 | 10.28 |
| **1-(R,R)** | 125 | 110 | 128 | 0 | 0 | 0 | 0.00 | 0.00 | 0.00 |
| **1-(R,S)** | 94 | 133 | 88 | 9 | 12 | 7 | 9.57 | 9.02 | 7.95 |
| Compou nd **1'** | 142 | 87 | 103 | 0 | 0 | 1 | 0.00 | 0.00 | 0.97 |
| **1'-(S,S)** | 78 | 123 | 106 | 11 | 18 | 17 | 14.10 | 14.63 | 16.04 |
| **1'-(S,R)** | 99 | 135 | 121 | 0 | 0 | 0 | 0.00 | 0.00 | 0.00 |

(continued)

| Treatme nt | Basic number of eggs before insecticide delivery | | | Number of hatched larvae 5 days after insecticide delivery | | | Hatching rate (%) | | |
|---|---|---|---|---|---|---|---|---|---|
| | Replic ate 1 | Replic ate 2 | Replic ate 3 | Replicat e 1 | Replicat e2 | Replicate 3 | Repli cate 1 | Repli cate 2 | Repli cate 3 |
| **1'-(R,R)** | 146 | 86 | 105 | 18 | 11 | 15 | 12.68 | 12.79 | 14.29 |
| **1'-(R,S)** | 104 | 145 | 97 | 0 | 0 | 0 | 0.00 | 0.00 | 0.00 |
| **S-10** | 85 | 109 | 112 | 0 | 0 | 0 | 0.00 | 0.00 | 0.00 |
| **R-10** | 98 | 131 | 117 | 15 | 19 | 17 | 15.31 | 14.50 | 14.53 |
| **Compo und 10** | 101 | 92 | 109 | 1 | 0 | 0 | 0.99 | 0.00 | 0.00 |
| **S-10'** | 133 | 86 | 102 | 0 | 0 | 0 | 0.00 | 0.00 | 0.00 |
| **R-10'** | 122 | 93 | 136 | 17 | 14 | 19 | 13.93 | 15.05 | 13.97 |
| **Compo und 10'** | 118 | 129 | 91 | 0 | 2 | 0 | 0.00 | 1.55 | 0.00 |
| **Compo und 19** | 113 | 120 | 98 | 0 | 1 | 0 | 0.00 | 0.83 | 0.00 |
| **19-(S,S)** | 125 | 108 | 96 | 18 | 15 | 11 | 14.4 | 13.89 | 11.46 |
| **19-(S,R)** | 117 | 92 | 134 | 0 | 0 | 0 | 0.00 | 0.00 | 0.00 |
| **19-(R,R )** | 95 | 104 | 115 | 12 | 14 | 15 | 12.63 | 13.46 | 13.04 |
| **19-(R,S)** | 87 | 125 | 109 | 0 | 0 | 0 | 0.00 | 0.00 | 0.00 |
| **Compo und 19'** | 105 | 87 | 135 | 1 | 1 | 0 | 0.95 | 1.15 | 0.00 |
| **19'-(S,S )** | 114 | 132 | 97 | 0 | 0 | 0 | 0.00 | 0.00 | 0.00 |
| **19'-(S,R )** | 117 | 126 | 84 | 11 | 13 | 8 | 9.40 | 10.32 | 9.52 |
| **19'-(R, R)** | 141 | 86 | 92 | 0 | 0 | 0 | 0.00 | 0.00 | 0.00 |
| **19'-(R,S )** | 133 | 98 | 103 | 12 | 9 | 9 | 9.02 | 9.18 | 8.74 |
| Solvent control | 129 | 93 | 117 | 124 | 92 | 113 | 97.21 | | |
| Clear water | 118 | 96 | 93 | 117 | 94 | 91 | 98.31 | | |

**Test Example 3 Effect on Hatching of Eggs of *Tetranychus urticae* by Compounds 1-(S,S), 1-(S,R), 1-(R,R), 1-(R,S), 1'-(S,S), 1'-(S,R), 1'-(R,R), 1'-(R,S), Compound 1, Compound 1', S-10, R-10, S-10', R-10' , Compound 10, Compound 10', Compound 19-(S,S), 19-(S,R), 19-(R,R), 19-(R,S), 19'-(S,S), 19'-(S,R), 19'-(R,R), 19'-(R,S), Compound 19, and Compound 19'**

This test adopted the method in Test Example 1 of technical section, mainly used leaf disc method to explore the inhibitory effect on hatching of eggs of *Tetranychus urticae* by compounds **1-(S,S), 1-(S,R), 1-(R,R), 1-(R,S), 1'-(S,S), 1'-(S,R), 1'-(R,R), 1'-(R,S), compound 1, compound 1' , S-10, R-10, S-10', R-10', compound 10, compound 10', compound 19-(S,S), 19-(S,R), 19-(R,R), 19-(R,S), 19'-(S,S), 19'-(S,R), 19'-(R,R), 19'-(R,S), compound 19, and compound 19'.** The concentration of all compounds was 5 ppm.

The experiments results are shown in Table 5, compounds **1-(S,S), 1-(R,R), 1'-(S, R), 1'-(R,S), compound 1, compound 1', S-10, S-10', compound 10, compound 10', 19-(S, R), 19-(R,S), 19'-(S,S), 19'-(R,R),** compound **19, and** compound **19'** all showed strong inhibitory activities on the hatching of eggs of *Tetranychus urticae* at 5 ppm.

Table 5

| Treatment | Basic number of eggs before insecticide delivery | | Number of hatched larvae 5 days after insecticide delivery | | Hatching rate (%) | |
|---|---|---|---|---|---|---|
| | Replicate 1 | Replicate 2 | Replicate 1 | Replicate 2 | Replicate 1 | Replicate 2 |
| Compound 1 | 106 | 77 | 1 | 1 | 0.94 | 1.30 |
| **1-(S,S)** | 83 | 76 | 0 | 0 | 0.00 | 0.00 |
| **1-(S,R)** | 92 | 74 | 10 | 9 | 10.87 | 12.16 |
| **1-(R,R)** | 103 | 85 | 0 | 0 | 0.00 | 0.00 |
| **1-(R,S)** | 78 | 63 | 11 | 88 | 14.10 | 12.70 |
| **Compound 1'** | 64 | 101 | 0 | 2 | 0.00 | 1.98 |
| **1'-(S,S)** | 63 | 74 | 13 | 12 | 20.63 | 16.22 |
| **1'-(S,R)** | 95 | 68 | 0 | 0 | 0.00 | 0.00 |
| **1'-(R,R)** | 67 | 83 | 12 | 16 | 17.91 | 19.28 |
| **1'-(R,S)** | 91 | 69 | 0 | 0 | 0.00 | 0.00 |
| **S-10** | 88 | 76 | 0 | 0 | 0.00 | 0.00 |
| **R-10** | 72 | 93 | 13 | 15 | 18.06 | 16.13 |
| **Compound 10** | 103 | 74 | 2 | 1 | 1.94 | 1.35 |
| **S-10'** | 69 | 85 | 0 | 0 | 0.00 | 0.00 |
| **R-10'** | 79 | 96 | 14 | 16 | 17.72 | 16.67 |
| **Compound 10'** | 85 | 91 | 1 | 1 | 1.18 | 1.10 |
| **Compound 19** | 87 | 92 | 0 | 1 | 0.00 | 1.09 |
| **19-(S,S)** | 103 | 95 | 18 | 14 | 17.48 | 14.74 |
| **19-(S,R)** | 94 | 98 | 0 | 0 | 0.00 | 0.00 |
| **19-(R,R)** | 89 | 106 | 16 | 19 | 17.98 | 17.92 |
| **19-(R,S)** | 78 | 96 | 0 | 0 | 0.00 | 0.00 |
| **Compound 19'** | 88 | 104 | 1 | 2 | 1.14 | 1.92 |
| **19'-(S,S)** | 100 | 99 | 0 | 0 | 0.00 | 0.00 |
| **19'-(S,R)** | 111 | 87 | 12 | 10 | 10.81 | 11.49 |
| **19'-(R,R)** | 95 | 98 | 0 | 0 | 0.00 | 0.00 |
| **19'-(R,S)** | 79 | 83 | 10 | 11 | 12.66 | 13.25 |
| Solvent control | 59 | 76 | 56 | 74 | 92.29 | |
| Clear water | 74 | 82 | 73 | 80 | 98.11 | |

**Test Example 4 Effect on Hatching of Eggs of *Panonychus citri* by Compounds 1-(S,S), 1-(S,R), 1-(R,R), 1-(R,S), 1'-(S,S), 1'-(S,R), 1'-(R,R), 1'-(R,S), Compound 1, Compound 1', S-10, R-10, S-10', R-10', Compound 10, Compound 10', Compound 19-(S,S), 19-(S,R), 19-(R,R), 19-(R,S), 19'-(S,S), 19'-(S,R), 19'-(R,R), 19'-(R,S), Compound 19, and Compound 19'.**

This test adopted the method in Test Example 1 of technical section, mainly used leaf disc method to explore the effect on hatching of eggs of *Panonychus citri* by compounds **1-(S,S), 1-(S,R), 1-(R,R), 1-(R,S), 1'-(S,S), 1'-(S,R), 1'-(R,R), 1'-(R,S), compound 1, compound 1', S-10, R-10, S-10', R-10', compound 10, compound 10', compound 19-(S,S), 19-(S,R), 19-(R,R), 19-(R,S), 19'-(S,S), 19'-(S,R), 19'-(R,R), 19'-(R,S), compound 19, and compound 19'**.The concentration of all compounds was 1 ppm.

The experiments results are shown in Table 6, compounds **1-(S,S), 1-(R,R), 1'-(S, R), 1'-(R,S),** compound 1 and compound **1' , S-10, S-10', compound 10, compound 10', 19-(S, R), 19-(R,S), 19'-(S,S), 19'-(R,R),** compound **19, and**

compound **19'** all showed good inhibitory effects on the hatching of eggs of *Panonychus citri* at 1 ppm.

Table 6

| Treatm ent | Basic number of eggs before insecticide delivery | | | Number of hatched larvae 5 days after insecticide delivery | | | Hatching rate (%) | | |
|---|---|---|---|---|---|---|---|---|---|
| | Replic ate 1 | Replic ate 2 | Replic ate 3 | Replicat e 1 | Replic ate 2 | Replic ate 3 | Replic ate 1 | Replic ate 2 | Repli cate 3 |
| Compo und 1 | 122 | 87 | 133 | 0 | 0 | 0 | 0.00 | 0.00 | 0.00 |
| **1-(S,S)** | 142 | 98 | 77 | 0 | 0 | 0 | 0.00 | 0.00 | 0.00 |
| **1-(S,R)** | 103 | 87 | 132 | 6 | 5 | 8 | 5.83 | 5.75 | 6.06 |
| **1-(R,R)** | 131 | 102 | 89 | 0 | 0 | 0 | 0.00 | 0.00 | 0.00 |
| **1-(R,S)** | 98 | 143 | 86 | 7 | 12 | 7 | 7.14 | 8.39 | 8.14 |
| **Compo und 1'** | 107 | 132 | 78 | 1 | 0 | 0 | 0.93 | 0.00 | 0.00 |
| **1'-(S,S)** | 129 | 81 | 108 | 19 | 13 | 18 | 14.73 | 16.05 | 16.67 |
| **1'-(S,R )** | 104 | 133 | 80 | 0 | 0 | 0 | 0.00 | 0.00 | 0.00 |
| **1'-(R,R )** | 72 | 99 | 127 | 6 | 7 | 7 | 8.33 | 7.07 | 5.51 |
| **1'-(R,S )** | 132 | 94 | 107 | 0 | 0 | 0 | 0.00 | 0.00 | 0.00 |
| **S-10** | 123 | 109 | 96 | 0 | 0 | 0 | 0.00 | 0.00 | 0.00 |
| **R-10** | 114 | 92 | 135 | 11 | 9 | 14 | 9.65 | 9.78 | 10.37 |
| **Compo und 10** | 99 | 81 | 117 | 1 | 0 | 0 | 1.01 | 0.00 | 0.00 |
| **S-10'** | 89 | 125 | 106 | 0 | 0 | 0 | 0.00 | 0.00 | 0.00 |
| **R-10'** | 136 | 97 | 104 | 14 | 10 | 10 | 10.29 | 10.31 | 9.62 |
| **Compo und 10'** | 112 | 137 | 109 | 0 | 1 | 1 | 0.00 | 0.73 | 0.92 |
| **Compo und** 19 | 125 | 106 | 94 | 0 | 0 | 0 | 0.00 | 0.00 | 0.00 |
| **19-(S,S )** | 119 | 101 | 86 | 12 | 10 | 9 | 10.08 | 9.90 | 10.47 |
| **19-(S,R )** | 102 | 98 | 125 | 0 | 0 | 0 | 0.00 | 0.00 | 0.00 |
| **19-(R, R)** | 133 | 107 | 84 | 10 | 9 | 7 | 7.52 | 8.41 | 8.33 |
| **19-(R,S )** | 92 | 123 | 114 | 0 | 0 | 0 | 0.00 | 0.00 | 0.00 |
| **Compo und 19'** | 89 | 98 | 116 | 0 | 0 | 0 | 0.00 | 0.00 | 0.00 |
| **19'-(S, S)** | 127 | 113 | 98 | 0 | 0 | 0 | 0.00 | 0.00 | 0.00 |
| **19'-(S, R)** | 94 | 107 | 119 | 9 | 11 | 12 | 9.57 | 10.28 | 10.08 |
| **19'-(R, R)** | 132 | 79 | 108 | 0 | 0 | 0 | 0.00 | 0.00 | 0.00 |
| **19'-(R, S)** | 115 | 96 | 94 | 12 | 10 | 9 | 10.43 | 10.42 | 9.57 |
| Solvent control | 103 | 101 | 107 | 98 | 97 | 103 | 95.82 | | |
| Clear water | 99 | 102 | 98 | 96 | 99 | 96 | 97.33 | | |

**Test Example 5 Effect on Hatching of Eggs of *Aleyrodidae* by Compounds 1-(S,S), 1-(S,R), 1-(R,R), 1-(R,S), 1'-(S,S), 1'-(S,R), 1'-(R,R), 1'-(R,S), Compound 1, Compound 1' , Compound 1', S-10, R-10, S-10', R-10' , Compound 10, Compound 10', Compound 19-(S,S), 19-(S,R), 19-(R,R), 19-(R,S), 19'-(S,S), 19'-(S,R), 19'-(R,R), 19'-(R,S), Compound 19, and Compound 19'.**

This test adopted the method in Test Example 1 of technical section, mainly used leaf disc method to explore the inhibitory effect on hatching of eggs of *Aleyrodidae* by compounds **1-(S,S), 1-(S,R), 1-(R,R), 1-(R,S), 1'-(S,S), 1'-(S,R), 1'-(R,R), 1'-(R,S), compound 1, compound 1'** , **S-10, R-10, S-10', R-10', compound 10, compound 10', compound 19-(S,S), 19-(S,R), 19-(R,R), 19-(R,S), 19'-(S,S), 19'-(S,R), 19'-(R,R), 19'-(R,S), compound 19, and compound**

**19'**.The concentration of all compounds was 10 ppm.

The experiments results are shown in Table 7, compounds **1-(S,S), 1-(R,R), 1'-(S, R), 1'-(R,S), compound 1, compound 1', S-10, S-10', compound 10, compound 10', 19-(S, R), 19-(R,S), 19'-(S,S), 19'-(R,R),** compound **19, and** compound **19'** all showed strong inhibitory activities on the hatching of eggs of *Aleyrodidae* at 10 ppm.

Table 7

| Treatm ent | Basic number of eggs before insecticide delivery | | | Number of hatched larvae 5 days after insecticide delivery | | | Hatching rate (%) | | |
|---|---|---|---|---|---|---|---|---|---|
| | Replic ate 1 | Replic ate 2 | Replic ate 3 | Replicat e 1 | Replic ate 2 | Replic ate 3 | Replic ate 1 | Replic ate 2 | Repli cate 3 |
| Compo und 1 | 89 | 133 | 112 | 0 | 2 | 0 | 0.00 | 1.50 | 0.00 |
| **1-(S,S)** | 87 | 96 | 131 | 0 | 0 | 0 | 0.00 | 0.00 | 0.00 |
| **1-(S,R)** | 114 | 128 | 105 | 11 | 12 | 11 | 9.65 | 9.38 | 10.48 |
| **1-(R,R)** | 149 | 121 | 94 | 0 | 0 | 0 | 0.00 | 0.00 | 0.00 |
| **1-(R,S)** | 88 | 135 | 121 | 7 | 10 | 9 | 7.95 | 7.41 | 7.44 |
| **Compo und 1'** | 107 | 79 | 88 | 2 | 0 | 1 | 1.87 | 0.00 | 1.14 |
| **1'-(S,S)** | 132 | 105 | 78 | 20 | 17 | 12 | 15.15 | 16.19 | 15.38 |
| **1'-(S,R )** | 94 | 141 | 89 | 0 | 0 | 0 | 0.00 | 0.00 | 0.00 |
| **1'-(R,R )** | 129 | 91 | 106 | 18 | 14 | 14 | 13.95 | 15.38 | 13.21 |
| **1'-(R,S )** | 110 | 96 | 128 | 0 | 0 | 0 | 0.00 | 0.00 | 0.00 |
| **S-10** | 102 | 92 | 104 | 0 | 0 | 0 | 0.00 | 0.00 | 0.00 |
| **R-10** | 143 | 138 | 121 | 14 | 13 | 12 | 9.79 | 9.42 | 9.92 |
| **Compo und 10** | 132 | 107 | 124 | 1 | 0 | 2 | 0.76 | 0.00 | 1.61 |
| **S-10'** | 81 | 128 | 136 | 0 | 0 | 0 | 0.00 | 0.00 | 0.00 |
| **R-10'** | 133 | 88 | 128 | 13 | 9 | 13 | 9.77 | 10.23 | 10.16 |
| **Compo und 10'** | 99 | 103 | 95 | 2 | 1 | 1 | 2.02 | 0.97 | 1.05 |
| **Compo und** 19 | 96 | 112 | 109 | 0 | 0 | 0 | 0.00 | 0.00 | 0.00 |
| **19-(S,S** ) | 89 | 133 | 114 | 11 | 16 | 14 | 12.36 | 12.03 | 12.28 |
| **19-(S,R )** | 78 | 102 | 95 | 0 | 0 | 0 | 0.00 | 0.00 | 0.00 |
| **19-(R, R)** | 92 | 98 | 125 | 11 | 10 | 14 | 11.96 | 10.20 | 11.20 |
| **19-(R,S)** | 102 | 113 | 108 | 0 | 0 | 0 | 0.00 | 0.00 | 0.00 |
| **Compo und 19'** | 115 | 86 | 99 | 0 | 0 | 0 | 0.00 | 0.00 | 0.00 |
| **19'-(S, S)** | 107 | 126 | 134 | 0 | 0 | 0 | 0.00 | 0.00 | 0.00 |
| **19'-(S, R)** | 95 | 127 | 98 | 10 | 13 | 10 | 10.53 | 10.24 | 10.20 |
| **19'-(R, R)** | 84 | 93 | 135 | 0 | 0 | 0 | 0.00 | 0.00 | 0.00 |
| **19'-(R, S)** | 126 | 117 | 87 | 13 | 11 | 10 | 10.32 | 9.40 | 11.49 |
| Solvent control | 129 | 93 | 112 | 127 | 90 | 109 | 97.51 | | |
| Clear water | 118 | 91 | 86 | 117 | 89 | 85 | 98.85 | | |

**Test Example 6 Effect on Hatching of Eggs of *Spodoptera litura* by Compounds 1-(S,S), 1-(S,R), 1-(R,R), 1-(R,S), 1'-(S,S), 1'-(S,R), 1'-(R,R), 1'-(R,S), Compound 1 , Compound 1', S-10, R-10, S-10', R-10' , Compound 10, Compound 10', Compounds 19-(S,S), 19-(S,R), 19-(R,R), 19-(R,S), 19'-(S,S), 19'-(S,R), 19'-(R,R), 19'-(R,S), Compound 19 , and Compound 19'.**

1. Experimental Solution

(1) Preparation of paper containing eggs: the egg mass was cut into small pieces, each containing about 60 eggs, soaked in a preformulated test agent for 10 minutes together with paper for egg raising (concentration of each compound of 500 ppm). After taken out, absorbent paper was used to absorb the excess solution adhered to the paper for egg raising and egg pieces.
(2) Cultivation: Each egg piece was separately placed in a glass test tube (5.0 cm in height, 2.5 cm in diameter, the same below), which was sealed with plastic paper punctured with fine holes using insect needles and cultivated in an artificial climate chamber at a temperature of (24 ± 1)°C, a relative humidity of (80 ± 10)%, and a photoperiod of L: D=12:12. When the eggs developed until they were about to hatch, castor leaves with a diameter of about 3 cm were added for feeding the hatching larvae;
(3) Observation: the number of hatched and unhatched eggs per egg piece was checked and recorded, and the hatching rate of eggs was calculated according to the formula. Each treatment was repeated for 3 times.
(4) Survey of results: The experimental materials of each treatment group were regularly hydrated and moisturized, and the hatching of eggs was observed. On the 4th day after insecticide delivery, the number of hatched eggs for each treatment was recorded, and the survey of results was recorded in the original notebook. According to the experimental requirements and characteristics of agents, the survey time can be shortened or extended.

Survey indicators:

1) Surveying and recording the number of hatched eggs for each treatment.
2) Recording the developmental status of experimental eggs of *Spodoptera litura,* behavior status of nymphs, including abnormal phenomena such as delayed or stopped development of eggs of *Spodoptera litura,* etc.
(5) Calculation method: Based on the survey data, the prevention and control effect of each treatment was calculated according to the following formula, and the calculation results were kept to two decimal places.

Egg hatching rate (%)=number of hatched eggs/total number of treated eggs*100

Prevention and control effect (%)=(hatching rate of eggs in control area - hatching rate of eggs in treatment area)/ hatching rate of eggs in control area)* 100

2. Experimental Results

The experiments results are shown in Table 8, compounds **1-(S,S), 1-(R,R), 1'-(S, R), 1'-(R,S)** , **S-10, S-10', 19-(S, R), 19-(R,S), 19'-(S,S), 19'-(R,R)** all showed good inhibitory activities on the hatching of eggs of *Spodoptera litura* at 500 ppm.

Table 8

| Treatme nt | Basic number of eggs before insecticide delivery | | | Number of unhatched larvae 4 days after insecticide delivery | | | Hatching rate (%) | | |
|---|---|---|---|---|---|---|---|---|---|
| | Replicat e 1 | Replicat e 2 | Replicat e 3 | Replicate 1 | Replicate 2 | Replicate 3 | Replic ate 1 | Replic ate 2 | Replic ate 3 |
| Compou nd 1 | 58 | 63 | 62 | 34 | 38 | 37 | 41.38 | 39.68 | 40.42 |
| 1-(S,S) | 59 | 65 | 58 | 45 | 48 | 46 | 23.73 | 26.15 | 20.69 |
| 1-(S,R) | 61 | 65 | 58 | 23 | 24 | 23 | 62.30 | 63.08 | 60.34 |
| 1-(R,R) | 63 | 57 | 59 | 47 | 45 | 45 | 22.39 | 21.05 | 23.73 |
| 1-(R,S) | 67 | 57 | 58 | 25 | 22 | 23 | 64.18 | 61.40 | 60.34 |
| Compou nd 1' | 59 | 62 | 64 | 35 | 37 | 39 | 40.68 | 40.32 | 39.06 |

(continued)

| Treatme nt | Basic number of eggs before insecticide delivery | | | Number of unhatched larvae 4 days after insecticide delivery | | | Hatching rate (%) | | |
|---|---|---|---|---|---|---|---|---|---|
| | Replicat e 1 | Replicat e 2 | Replicat e 3 | Replicate 1 | Replicate 2 | Replicate 3 | Replic ate 1 | Replic ate 2 | Replic ate 3 |
| 1'-(S,S) | 62 | 58 | 56 | 22 | 21 | 19 | 64.52 | 63.79 | 66.07 |
| 1'-(S,R) | 61 | 63 | 57 | 44 | 46 | 45 | 27.87 | 26.98 | 21.05 |
| 1'-(R,R) | 55 | 56 | 59 | 21 | 20 | 22 | 61.82 | 64.29 | 62.71 |
| 1'-(R,S) | 60 | 64 | 59 | 43 | 48 | 47 | 28.33 | 25.00 | 20.34 |
| S-10 | 56 | 70 | 62 | 44 | 54 | 47 | 21.43 | 22.86 | 24.19 |
| R-10 | 69 | 53 | 58 | 27 | 19 | 22 | 60.87 | 64.15 | 62.07 |
| Compou nd 10 | 63 | 61 | 57 | 37 | 36 | 33 | 41.27 | 40.98 | 42.11 |
| S-10' | 59 | 64 | 60 | 46 | 50 | 47 | 22.03 | 21.88 | 21.67 |
| R-10' | 55 | 58 | 66 | 21 | 22 | 24 | 61.82 | 62.07 | 63.64 |
| Compou nd 10' | 64 | 62 | 59 | 38 | 36 | 34 | 40.63 | 41.94 | 42.37 |
| Compou nd 19 | 51 | 63 | 57 | 36 | 44 | 40 | 29.41 | 30.16 | 29.82 |
| 19-(S,S) | 60 | 58 | 55 | 30 | 28 | 27 | 50.00 | 51.72 | 50.91 |
| 19-(S,R) | 57 | 65 | 52 | 48 | 55 | 44 | 15.79 | 15.38 | 15.38 |
| 19-(R,R) | 59 | 62 | 63 | 29 | 30 | 31 | 50.85 | 51.61 | 50.79 |
| 19-(R,S) | 64 | 59 | 60 | 54 | 50 | 50 | 15.63 | 15.25 | 16.67 |
| Compou nd 19' | 58 | 56 | 64 | 39 | 38 | 41 | 32.76 | 32.14 | 35.94 |
| 19'-(S,S) | 57 | 66 | 53 | 47 | 53 | 43 | 17.54 | 19.70 | 18.87 |
| 19'-(S,R) | 56 | 62 | 67 | 26 | 29 | 31 | 53.57 | 53.23 | 53.73 |
| 19'-(R,R) | 57 | 59 | 68 | 47 | 49 | 55 | 17.54 | 16.95 | 19.12 |
| 19'-(R,S) | 61 | 65 | 54 | 30 | 32 | 27 | 51.67 | 50.77 | 50.00 |
| Solvent control | 58 | 61 | 63 | 54 | 57 | 58 | 92.87 | | |
| Clear water | 57 | 62 | 59 | 55 | 60 | 56 | 96.06 | | |

**Test Example 7 Effect on Hatching of Eggs of *Harmonia axyridis* by Compounds 1-(S,S), 1-(S,R), 1-(R,R), 1-(R,S), 1'-(S,S), 1'-(S,R), 1'-(R,R), 1'-(R,S), Compound 1, Compound 1', S-10, R-10, S-10', R-10', Compound 10, Compound 10', Compound 19-(S,S ) , 19-(S,R), 19-(R,R), 19-(R,S), 19'-(S,S), 19'-(S,R), 19'-(R,R), 19'-(R,S), Compound 19, and Compound 19'.**

1. Experimental Solution

The eggs of *Harmonia axyridis* cards (with approximately 20 eggs per card) were purchased from Henan Jiyuan Baiyun Industry Co., Ltd. in Henan Province. The number of eggs on the egg cards was counted as the basic number before insecticide delivery, and 5 cards were used as one treatment, with 3 replicates per treatment. After immersing the egg cards in a 100 ppm insecticide solution for 30 seconds, they were taken out and air dried, then cultivated under moisturizing conditions, and the treated mite eggs and leaf butterflies were cultivated at 27°C. 4 days after insecticide delivery, the hatching status of eggs of *Harmonia axyridis* was investigated, and the prevention and control effect was calculated according to the following formula.

Egg hatching rate (%)=number of hatched eggs/total number of treated eggs*100

Prevention and control effect (%)=(hatching rate of eggs in control area - hatching rate of eggs in treatment area)/ hatching rate of eggs in control area)* 100

2. Experimental Results

The experiments results are shown in Table 9, compounds **1-(S,S), 1-(R,R), 1'-(S, R), 1'-(R,S), compound 1** , **compound 1'** , **S-10, S-10'** , **compound 10, compound 10', 19-(S, R), 19-(R,S), 19'-(S,S), 19'-(R,R)** , compound **19, and** compound **19'** all showed strong inhibitory activities on the hatching of eggs of *Harmonia axyridis* at 100 ppm.

**Table 9**

| Treatm ent | Basic number of eggs before insecticide delivery | | | Number of hatched larvae 4 days after insecticide delivery | | | Hatching rate (%) | | |
|---|---|---|---|---|---|---|---|---|---|
| | Replic ate 1 | Replic ate 2 | Replic ate 3 | Replic ate 1 | Replicat e2 | Replic ate 3 | Replic ate 1 | Replic ate 2 | Repli cate 3 |
| Compo und 1 | 112 | 94 | 99 | 7 | 6 | 6 | 6.25 | 6.38 | 6.06 |
| **1-(S,S)** | 106 | 88 | 93 | 0 | 0 | 0 | 0.00 | 0.00 | 0.00 |
| **1-(S,R)** | 108 | 99 | 102 | 13 | 14 | 14 | 12.04 | 14.14 | 13.73 |
| **1-(R,**R) | 92 | 110 | 101 | 0 | 0 | 0 | 0.00 | 0.00 | 0.00 |
| **1-(R,S)** | 111 | 88 | 95 | 11 | 9 | 10 | 9.91 | 10.23 | 10.53 |
| **Compo und 1'** | 102 | 96 | 106 | 6 | 4 | 7 | 5.88 | 4.17 | 6.60 |
| **1'-(S,S)** | 92 | 98 | 107 | 18 | 19 | 21 | 19.57 | 19.39 | 18.69 |
| **1'-(S,R)** | 101 | 105 | 94 | 0 | 0 | 0 | 0.00 | 0.00 | 0.00 |
| **1'-(R,R )** | 88 | 95 | 111 | 15 | 16 | 18 | 17.05 | 16.84 | 16.22 |
| **1'-(R,S )** | 93 | 87 | 109 | 0 | 0 | 0 | 0.00 | 0.00 | 0.00 |
| **S-10** | 112 | 98 | 136 | 0 | 0 | 0 | 0.00 | 0.00 | 0.00 |
| **R-10** | 125 | 84 | 101 | 15 | 10 | 13 | 12.00 | 11.90 | 12.87 |
| **Compo und 10** | 99 | 78 | 126 | 4 | 3 | 7 | 4.04 | 3.85 | 5.56 |
| **S-10'** | 94 | 107 | 116 | 0 | 0 | 0 | 0.00 | 0.00 | 0.00 |
| **R-10'** | 133 | 102 | 94 | 17 | 14 | 12 | 12.78 | 13.73 | 12.77 |
| **Compo und 10'** | 87 | 95 | 122 | 5 | 5 | 6 | 5.75 | 5.26 | 4.92 |
| Compo und 19 | 92 | 110 | 96 | 3 | 5 | 4 | 3.26 | 4.45 | 4.17 |
| **19-(S,S )** | 95 | 103 | 104 | 13 | 15 | 16 | 13.68 | 14.56 | 15.38 |
| **19-(S,R )** | 107 | 100 | 98 | 0 | 0 | 0 | 0.00 | 0.00 | 0.00 |
| **19-(R, R)** | 97 | 104 | 102 | 14 | 15 | 14 | 14.43 | 14.42 | 13.73 |
| **19-(R,S )** | 102 | 105 | 98 | 0 | 0 | 0 | 0.00 | 0.00 | 0.00 |
| **Compo und 19'** | 89 | 96 | 108 | 4 | 5 | 7 | 4.49 | 5.21 | 6.48 |
| **19'-(S, S)** | 95 | 97 | 103 | 0 | 0 | 0 | 0.00 | 0.00 | 0.00 |
| **19'-(S, R)** | 96 | 107 | 112 | 10 | 10 | 11 | 10.42 | 9.35 | 9.82 |
| **19'-(R, R)** | 112 | 104 | 94 | 0 | 0 | 0 | 0.00 | 0.00 | 0.00 |
| **19'-(R, S)** | 107 | 95 | 92 | 11 | 10 | 9 | 10.28 | 10.53 | 9.78 |
| Solvent control | 89 | 112 | 91 | 86 | 107 | 87 | 95.92 | | |
| Clear water | 95 | 107 | 104 | 92 | 104 | 100 | 96.73 | | |

**Test Example 8 Preliminary Screening on *Antifungal*(*Magnaporthe Grisea)* Activity of Compounds 1-(S,S), 1-(S,R), 1-(R,R), 1-(R,S), 1'-(S,S), 1'-(S,R), 1'-(R,R), 1'-(R,S), Compound 1, Compound 1' , S-10, R-10, S-10', R-10' , Compound 10, Compound 10', Compound 19-(S,S), 19-(S,R), 19-(R,R), 19-(R,S), 19'-(S,S), 19'-(S,R), 19'-(R,R), 19'-(R,S), Compound 19, and Compound 19'.**

1. Experimental Solution

Experimental materials: culture medium, activated bacteria, sterile water, 96-well plate, and micro-discharge gun.

Quick screening system (200 μL): culture medium (150 μL)+insecticide (40 μL)+bacteria (10 μL).

Experimental steps: insecticide formulation, addition of culture medium, addition of bacteria, and detection

(1) Insecticide formulation: Insecticide was formulated according to a final concentration of 100 ppm, and the prepared insecticide was transferred into a 1.5 mL centrifuge tube for later use.
(2) The culture medium and insecticides were added into the prepared 96-well plate using a micro-discharge gun.
(3) Formulation of inoculum: The cultured culture dish (*Magnaporthe Grisea*) was taken out, 15 mL of sterile water was added to the culture dish, the surface of the mycelia was slided with the pipette tip to break the mycelia and dissolve them into the sterile water, 10 μL of bacterial suspension was taken out and placed on a glass slide for microscopic examination, ensuring that there were no less than 10 mycelia in the field of view. The bacteria were cultivated to OD600=1.0 and diluted 1000 folds to obtain the inoculum; the number of oomycete zoospores shall not be less than $1*10^5$/mL.
(4) Detection: Detection of fungi: OD=450nm, detection of bacteria:OD=600 nm, and they were recorded as 0-hour data. After cultivating for the corresponding times, the growth data were recorded and the antifungal rate was calculated according to the formula.

Antifungal rate (%)=((blank control OD (72h) - blank control OD (0h)) - (treatment OD (72h) - treatment OD (0h))/ blank control OD (72h) - blank control OD (0h))*100

2. Experimental results

The experiments results are shown in Table 10, compounds **1-(S,S), 1-(S,R), 1-(R,R), 1-(R,S), 1'-(S,S), 1'-(S,R), 1'-(R,R), 1'-(R,S), compound 1, compound 1' , S-10, R-10, S-10', R-10', compound 10, compound 10', compounds 19-(S,S), 19-(S,R), 19-(R,R), 19-(R,S), 19'-(S,S), 19'-(S,R), 19'-(R,R), 19'-(R,S), compound 19, and compound 19'** had a strong inhibitory rate against *Magnaporthe grisea* at 100 ppm, especially the antifungal rate of compounds **1-(S,S), 1-(R,R), 1'-(R,S), 1'-(S,R), compound 1, and compound 1' , S-10, S-10' , compound 10, compound 10', 19-(S, R), 19-(R,S), 19'-(S,S), 19'-(R,R),** compound **19, and** compound **19' can reach up to 90%** or higher.

**Table 10**

| Nos. | Antifungal rate of *Magnaporthe grisea* (%) | | | Average antifungal rate (%) |
|---|---|---|---|---|
| | Replicate 1 | Replicate 2 | Replicate 3 | |
| Compound 1 | 96.34 | 95.11 | 94.82 | 95.42 |
| **1-(S,S)** | 99.38 | 95.02 | 97.64 | 97.35 |
| **1-(S,R)** | 87.32 | 82.96 | 78.48 | 82.92 |
| **1-(R,R)** | 99.41 | 99.12 | 98.34 | 98.96 |
| **1-(R,S)** | 75.86 | 83.08 | 86.80 | 81.91 |
| **Compound 1'** | 95.78 | 98.76 | 88.30 | 94.28 |
| **1'-(S,S)** | 84.82 | 83.06 | 76.44 | 81.44 |
| **1'-(S,R)** | 95.80 | 102.01 | 99.85 | 99.22 |
| **1'-(R,R)** | 84.82 | 83.06 | 86.71 | 84.86 |
| **1'-(R,S)** | 100.26 | 99.74 | 100.26 | 100.09 |
| **S-10** | 98.63 | 99.72 | 97.51 | 98.62 |
| **R-10** | 81.97 | 83.68 | 81.92 | 82.52 |

(continued)

| Nos. | Antifungal rate of *Magnaporthe grisea* (%) | | | Average antifungal rate (%) |
|---|---|---|---|---|
| | Replicate 1 | Replicate 2 | Replicate 3 | |
| **Compound 10** | 93.89 | 94.52 | 94.36 | 94.26 |
| **S-10'** | 100.03 | 99.64 | 98.27 | 99.31 |
| **R-10'** | 82.71 | 81.95 | 83.14 | 82.60 |
| **Compound 10'** | 94.01 | 95.16 | 93.77 | 94.31 |
| Compound 19 | 97.23 | 95.14 | 94.35 | 95.57 |
| **19-(S,S)** | 86.52 | 83.17 | 84.38 | 84.69 |
| **19-(S,R)** | 100.10 | 99.34 | 99.87 | 99.77 |
| **19-(R,R)** | 81.29 | 83.56 | 83.45 | 82.77 |
| **19-(R,S)** | 99.73 | 100.15 | 100.24 | 100.04 |
| **Compound 19'** | 91.33 | 93.48 | 92.95 | 92.59 |
| **19'-(S,S)** | 97.41 | 96.58 | 97.29 | 97.09 |
| **19'-(S,R)** | 86.44 | 85.97 | 86.28 | 86.23 |
| **19'-(R,R)** | 96.97 | 97.35 | 97.26 | 97.19 |
| **19'-(R,S)** | 85.69 | 86.41 | 86.27 | 86.12 |

**Experimental Example 9 Effects of Sulfite Compounds on Hatching of Eggs of *Tetranychus cinnabarinus***

1. Experimental Method

This test adopted the method in Test Example 1 of technical section, mainly used leaf disc method to explore the effect on hatching of eggs of *Panonychus citri* by **compounds 1-30 and compounds 1'-30'.** The concentration of all compound was 100 ppm.

2. Experimental Results

The experiments results are shown in Table 11, the hatching rates by compound 1, compound 2, compound 3, compound 4, compound 5, compound 6, compound 7, compound 8, compound 9, compound 1', compound 2', compound 3', compound 4', compound 5', compound 6', compound 7', compound 8' and compound 9' were all below 15% , showing good activity in killing eggs of mites.

Table 11

| Treatm ent | Basic number of eggs before insecticide delivery | | | Unhatched number of eggs 7 days after insecticide delivery | | | Hatching rate (%) | | |
|---|---|---|---|---|---|---|---|---|---|
| | Replic ate 1 | Replic ate 2 | Replic ate 3 | Replic ate 1 | Replic ate 2 | Replicat e 3 | Replic ate 1 | Replic ate 2 | Repli cate 3 |
| Compo und 1 | 142 | 95 | 107 | 142 | 95 | 107 | 0.00 | 0.00 | 0.00 |
| Compo und 2 | 133 | 88 | 103 | 132 | 87 | 101 | 0.76 | 1.14 | 1.94 |
| Compo und 3 | 116 | 78 | 108 | 105 | 67 | 99 | 9.48 | 14.10 | 8.33 |
| Compo und 4 | 87 | 137 | 112 | 99 | 127 | 106 | 8.05 | 7.30 | 5.36 |
| Compo und 5 | 82 | 126 | 93 | 75 | 113 | 83 | 8.54 | 10.32 | 10.75 |
| Compo und 6 | 98 | 134 | 102 | 84 | 115 | 89 | 14.29 | 14.18 | 12.75 |
| Compo und 7 | 102 | 135 | 87 | 94 | 124 | 81 | 7.84 | 8.15 | 6.90 |
| Compo und 8 | 96 | 118 | 133 | 83 | 107 | 121 | 13.54 | 9.32 | 9.02 |

(continued)

| Treatm ent | Basic number of eggs before insecticide delivery | | | Unhatched number of eggs 7 days after insecticide delivery | | | Hatching rate (%) | | |
|---|---|---|---|---|---|---|---|---|---|
| | Replic ate 1 | Replic ate 2 | Replic ate 3 | Replic ate 1 | Replic ate 2 | Replicat e 3 | Replic ate 1 | Replic ate 2 | Repli cate 3 |
| Compo und 9 | 108 | 112 | 89 | 93 | 96 | 76 | 13.89 | 14.29 | 14.61 |
| Compo und 10 | 121 | 89 | 104 | 121 | 89 | 104 | 0.00 | 0.00 | 0.00 |
| Compo und 11 | 134 | 98 | 122 | 132 | 97 | 121 | 1.49 | 1.02 | 0.82 |
| Compo und 12 | 123 | 79 | 136 | 112 | 71 | 124 | 8.94 | 10.13 | 8.82 |
| Compo und 13 | 92 | 113 | 144 | 87 | 105 | 136 | 5.43 | 7.08 | 5.56 |
| Compo und 14 | 78 | 107 | 141 | 71 | 99 | 128 | 8.97 | 7.48 | 9.22 |
| Compo und 15 | 94 | 123 | 111 | 82 | 106 | 95 | 12.77 | 13.82 | 14.41 |
| Compo und 16 | 111 | 135 | 92 | 103 | 124 | 85 | 7.21 | 8.15 | 7.61 |
| Compo und 17 | 99 | 128 | 133 | 86 | 115 | 121 | 13.13 | 10.16 | 9.02 |
| Compo und 18 | 112 | 141 | 89 | 98 | 122 | 76 | 12.50 | 13.48 | 14.61 |
| Compo und 19 | 122 | 92 | 98 | 122 | 92 | 98 | 0.00 | 0.00 | 0.00 |
| Compo und 20 | 78 | 86 | 105 | 71 | 77 | 95 | 8.97 | 10.47 | 9.52 |
| Compo und 21 | 93 | 106 | 99 | 81 | 91 | 85 | 12.90 | 14.15 | 14.14 |
| Compo und 22 | 114 | 105 | 127 | 114 | 105 | 127 | 0.00 | 0.00 | 0.00 |
| Compo und 23 | 92 | 109 | 111 | 87 | 103 | 105 | 5.43 | 5.50 | 5.41 |
| Compo und 24 | 89 | 97 | 122 | 84 | 91 | 113 | 5.62 | 6.19 | 7.38 |
| Compo und 25 | 103 | 98 | 135 | 103 | 98 | 135 | 0.00 | 0.00 | 0.00 |
| Compo und 26 | 117 | 86 | 92 | 109 | 79 | 84 | 6.84 | 8.14 | 8.70 |
| Compo und 27 | 125 | 92 | 107 | 110 | 80 | 94 | 12.00 | 13.04 | 12.14 |
| Compo und 28 | 129 | 104 | 93 | 129 | 104 | 93 | 0.00 | 0.00 | 0.00 |
| Compo und 29 | 133 | 84 | 115 | 126 | 79 | 108 | 5.26 | 5.95 | 6.09 |
| Compo und 30 | 87 | 116 | 127 | 76 | 101 | 110 | 12.64 | 12.93 | 13.39 |
| Compo und 1’ | 138 | 86 | 112 | 138 | 86 | 112 | 0.00 | 0.00 | 0.00 |
| Compo und 2’ | 89 | 132 | 111 | 89 | 130 | 110 | 0.00 | 1.52 | 0.90 |
| Compo und 3’ | 123 | 129 | 76 | 112 | 117 | 68 | 8.94 | 9.30 | 10.53 |
| Compo und 4’ | 141 | 110 | 92 | 126 | 101 | 86 | 10.64 | 8.18 | 6.52 |
| Compo und 5’ | 84 | 98 | 133 | 76 | 88 | 119 | 9.52 | 10.20 | 10.53 |
| Compo und 6’ | 108 | 136 | 97 | 93 | 117 | 84 | 13.89 | 13.97 | 13.40 |
| Compo und 7’ | 115 | 107 | 144 | 106 | 98 | 134 | 7.83 | 8.41 | 6.94 |
| Compo und 8’ | 78 | 97 | 128 | 70 | 88 | 115 | 10.26 | 9.28 | 10.16 |
| Compo und 9’ | 92 | 115 | 132 | 79 | 98 | 112 | 14.13 | 14.78 | 15.15 |
| Compo und 10’ | 95 | 126 | 142 | 95 | 126 | 142 | 0.00 | 0.00 | 0.00 |
| Compo und 11’ | 87 | 123 | 111 | 87 | 121 | 110 | 0.00 | 1.63 | 0.90 |
| Compo und 12’ | 113 | 131 | 77 | 103 | 119 | 69 | 8.85 | 9.16 | 10.39 |
| Compo und 13’ | 131 | 100 | 95 | 117 | 92 | 86 | 10.69 | 8.00 | 9.47 |
| Compo und 14’ | 92 | 107 | 137 | 83 | 96 | 123 | 9.78 | 10.28 | 10.22 |
| Compo und 15’ | 118 | 136 | 95 | 112 | 117 | 82 | 13.55 | 13.97 | 13.68 |

(continued)

| Treatm ent | Basic number of eggs before insecticide delivery | | | Unhatched number of eggs 7 days after insecticide delivery | | | Hatching rate (%) | | |
|---|---|---|---|---|---|---|---|---|---|
| | Replic ate 1 | Replic ate 2 | Replic ate 3 | Replic ate 1 | Replic ate 2 | Replicat e 3 | Replic ate 1 | Replic ate 2 | Repli cate 3 |
| Compo und 16' | 122 | 131 | 114 | 112 | 119 | 103 | 8.20 | 9.16 | 9.65 |
| Compo und 17' | 79 | 99 | 128 | 70 | 90 | 115 | 11.39 | 9.09 | 10.16 |
| Compo und 18' | 93 | 115 | 141 | 80 | 98 | 122 | 13.98 | 14.78 | 13.48 |
| Compo und 19' | 108 | 119 | 91 | 108 | 119 | 91 | 0.00 | 0.00 | 0.00 |
| Compo und 20' | 140 | 106 | 83 | 127 | 97 | 76 | 9.29 | 8.49 | 8.43 |
| Compo und 21' | 88 | 95 | 114 | 77 | 83 | 100 | 12.50 | 12.63 | 12.28 |
| Compo und 22' | 134 | 91 | 110 | 134 | 91 | 110 | 0.00 | 0.00 | 0.00 |
| Compo und 23' | 117 | 98 | 126 | 110 | 93 | 117 | 5.98 | 6.12 | 7.14 |
| Compo und 24' | 112 | 119 | 98 | 103 | 111 | 90 | 8.04 | 6.72 | 8.16 |
| Solvent control | 94 | 85 | 106 | 6 | 2 | 8 | 94.57 | | |
| Clear water | 81 | 121 | 93 | 5 | 6 | 3 | 95.21 | | |

**Example 10**

The ginger rhizome extract and volatile oil of Kaempferia galanga L rhizome were used to prepare a mixture of ginger rhizome and Kaempferia galanga L rhizome at a compounding ratio of 7:3, which was then compounded with compound 1, compound 10 and compound 19, and a leaf -disc method was used to evaluate the control efficacy on eggs of *Tetranychus cinnabarinus* based on the Experimental method in Example 1.

The ginger rhizome extract was prepared by extracting the ginger rhizome using a mixed solvent of ethyl acetate and ethanol at a ratio of 1:4.

According to the concept of independent joint action proposed by Bliss, the theoretical mortality rate P when the pesticides and acaricides are used in mixture can be calculated using the following equation:

$$P = Pm + Pn(1 - Pm)$$

Pm is the mortality rate (%) of the target when the first active component is used at a concentration of m; and Pn is the mortality rate (%) of the target when the second active component is used at a concentration of n.

If the actual mortality rate of the target is greater than the theoretical mortality rate P after the two active components are mixed at a certain concentration, it is determined that the mixing of the two active components at the set concentration has a synergistic effect, on the contrary it is an antagonistic effect.

The experimental results are shown in Table 12, after the compound 1, compound 10, and compound 19 are compounded with a mixture of ginger rhizome and Kaempferia galanga L rhizome, it has a synergistic effect on controlling of eggs of *Tetranychus cinnabarinus.*

Table 12

| Nos. | Treatment | Concentrations (mg/L) | Control efficacy for eggs of *Tetranychus cinnabarinus* on day 5 (%) | Theoretical control efficacy (%) | Whether synergistic interaction or not |
|---|---|---|---|---|---|
| 1 | A mixture of gin-ger and Kaemp-feria galanga L (T1) | 500 | 35.38 | / | / |
| 2 | | 200 | 15.24 | / | / |

(continued)

| Nos. | Treatment | Concentrations (mg/L) | Control efficacy for eggs of *Tetranychus cinnabarinus* on day 5 (%) | Theoretical control efficacy (%) | Whether synergistic interaction or not |
|---|---|---|---|---|---|
| 3 | Compound 1 (T2) | 0.3 | 52.19 | / | / |
| 4 | | 0.2 | 33.58 | / | / |
| 5 | | 0.1 | 17.12 | / | / |
| 6 | Compound 10 (T3) | 0.5 | 68.12 | / | / |
| 7 | | 0.2 | 20.79 | / | / |
| 8 | | 0.1 | 11.45 | / | / |
| 9 | Compound 19 (T4) | 0.25 | 48.73 | / | / |
| 10 | | 0.2 | 36.14 | / | / |
| 11 | | 0.1 | 20.57 | / | / |
| 12 | T1:T2=500:0.3 | 500+0.3 | 76.21 | 69.11 | Yes |
| 13 | T1:T2=500:0.2 | 500+0.2 | 65.84 | 57.08 | Yes |
| 14 | T1:T2=500:0.1 | 500+0.1 | 52.11 | 46.44 | Yes |
| 15 | T1:T2=200:0.3 | 200+0.3 | 67.83 | 59.48 | Yes |
| 16 | T1:T2=200:0.2 | 200+0.2 | 51.26 | 43.70 | Yes |
| 17 | T1:T2=200:0.1 | 200+0.1 | 40.32 | 29.75 | Yes |
| 18 | T1:T3=500:0.5 | 500+0.5 | 85.74 | 79.40 | Yes |
| 19 | T1:T3=500:0.2 | 500+0.2 | 54.26 | 48.81 | Yes |
| 20 | T1:T3=500:0.1 | 500+0.1 | 48.15 | 42.78 | Yes |
| 21 | T1:T3=200:0.5 | 200+0.5 | 79.06 | 72.98 | Yes |
| 22 | T1:T3=200:0.2 | 200+0.2 | 40.18 | 32.86 | Yes |
| 23 | T1:T3=200:0.1 | 200+0.1 | 30.64 | 24.95 | Yes |
| 24 | T1:T4=500:0.25 | 500+0.25 | 73.12 | 66.87 | Yes |
| 25 | T1:T4=500:0.2 | 500+0.2 | 65.03 | 58.73 | Yes |
| 26 | T1:T4=500:0.1 | 500+0.1 | 52.44 | 48.67 | Yes |
| 27 | T1:T4=200:0.25 | 200+0.25 | 61.52 | 56.54 | Yes |
| 28 | T1:T4=200:0.2 | 200+0.2 | 50.19 | 45.87 | Yes |
| 29 | T1:T4=200:0.1 | 200+0.1 | 40.01 | 32.68 | Yes |

The above embodiments are only illustrative description of the principles and efficacy of the present disclosure, and are not intended to limit the present disclosure. Those skilled in the art may modify or alter the above embodiments without departing from the spirit and scope of the present disclosure. Therefore, all equivalent modifications or alterations made by those having ordinary knowledge in the relevant technical field without departing from the spirit and technical idea disclosed in the present disclosure should still be covered by the claims of the present disclosure.

## Claims

**1.** A sulfite compound having a structure as shown in formula (A) or a mesomer, a racemate, a stereisomer and a pharmaceutically acceptable salt thereof:

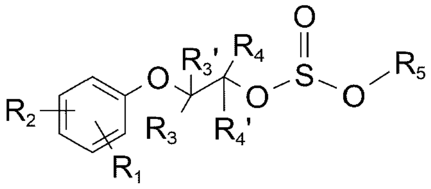

(A)

wherein, $R_1$ and $R_2$ are independently selected from hydrogen, halogen, a substituted or unsubstituted $C_1$-$C_{10}$ alkyl, a substituted or unsubstituted $C_1$-$C_{10}$ alkoxy, a $C_2$-$C_{10}$ alkoxycarbonyl, a $C_2$-$C_{10}$ alkylcarbonyl and a $C_1$-$C_{10}$ carbonyl;

$R_3$, $R_3$', $R_4$, and $R_4$' are each independently selected from hydrogen, $C_1$-$C_5$ alkyl, $C_1$-$C_5$ alkenyl; or, $R_3$, $R_3$', $R_4$, and $R_4$' together with the carbon atom to which they are attached form a five-membered heterocycloalkyl;

$R_5$ is selected from halogen, and a substituted or unsubstituted $C_1$-$C_{10}$ alkyl.

**2.** The sulfite compound or a mesomer, a racemate, a stereisomer or a pharmaceutically acceptable salt thereof according to claim **1,** wherein, the $C_1$-$C_5$ alkyl is selected from methyl and ethyl; the $C_1$-$C_5$ alkenyl is selected from ethenyl; and the five-membered heterocycloalkyl is selected from

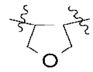

.

**3.** The sulfite compound or a mesomer, a racemate, a stereisomer or a pharmaceutically acceptable salt thereof according to claim 1, wherein, the structure of the compound is selected from one of the following:

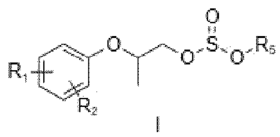

I

or

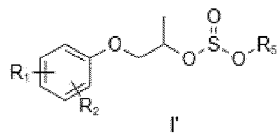

I'

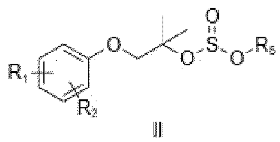

II

or

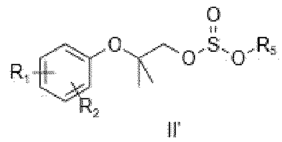

II'

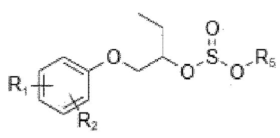

III

or

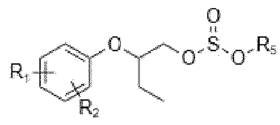

III'

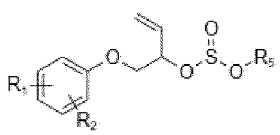

IV

or

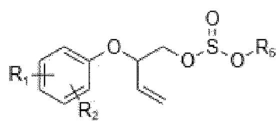

IV'

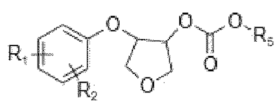

V

or

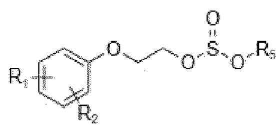

VI

**4.** The sulfite compound or a mesomer, a racemate, stereisomer or a pharmaceutically acceptable salt thereof according to claim 1, wherein, the $R_5$ is selected from fluoroethyl, bromoethyl, chloroethyl, 2,2-difluoroethyl, and 2,2-dichloroethyl; and $R_1$ and $R_2$ are independently selected from H, F, Cl, and Br.

**5.** The sulfite compound or a mesomer, a racemate, stereisomer or a pharmaceutically acceptable salt thereof according to claim 1, wherein, $R_5$ is $-CH_2CH_2F$; and $R_1$ and $R_2$ are Cl.

**6.** The sulfite compound or a mesomer, a racemate, a stereisomer and a pharmaceutically acceptable salt thereof according to claim 1, wherein the compound is selected from the following compounds:

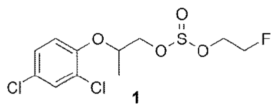
1
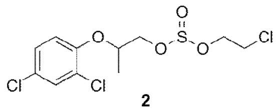
2
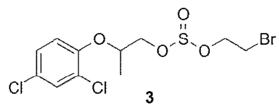
3
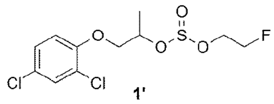
1'
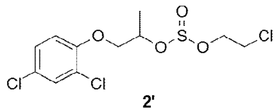
2'
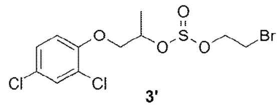
3'
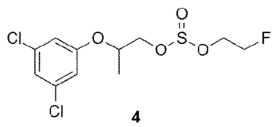
4
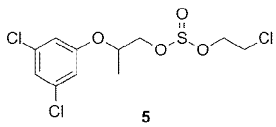
5
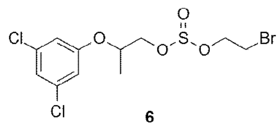
6
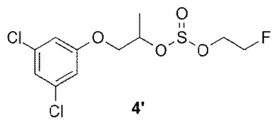
4'
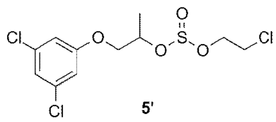
5'
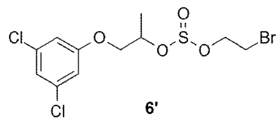
6'
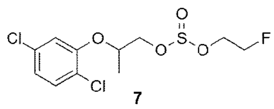
7
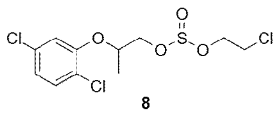
8
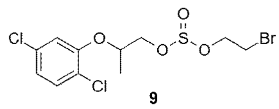
9
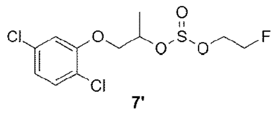
7'
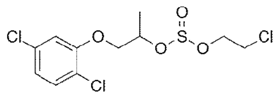
8'
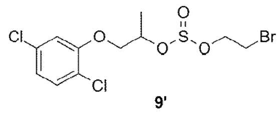
9'
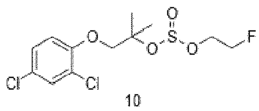
10
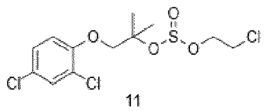
11
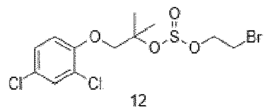
12
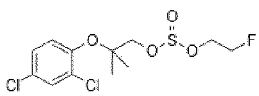
10'
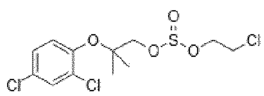
11'
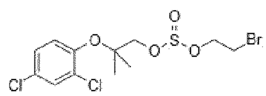
12'
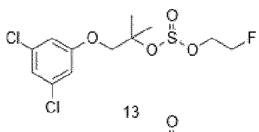
13
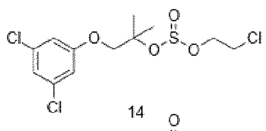
14
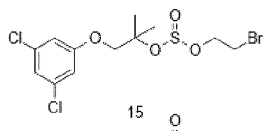
15
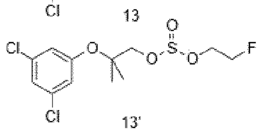
13'
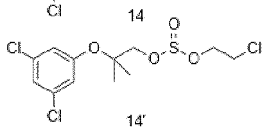
14'
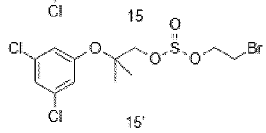
15'

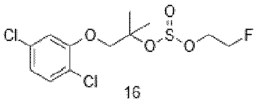
16
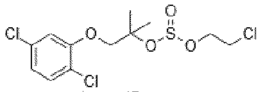
17
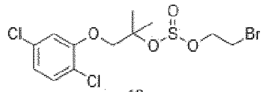
18
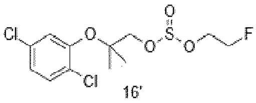
16'
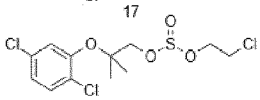
17'
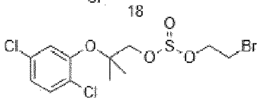
18'

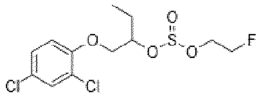
19
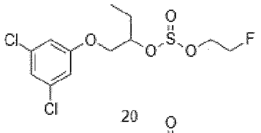
20
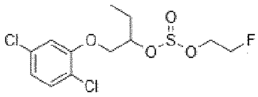
21
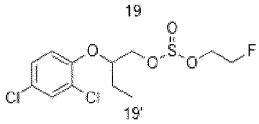
19'
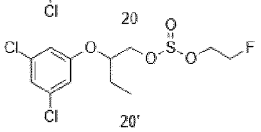
20'
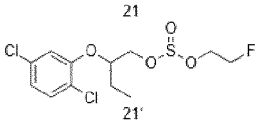
21'

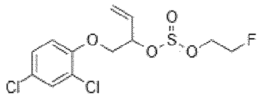
22
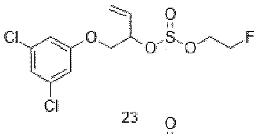
23
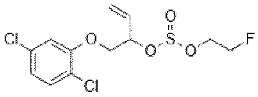
24
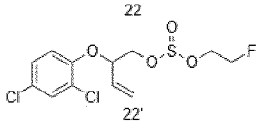
22'
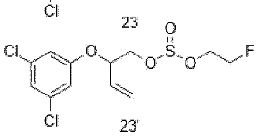
23'
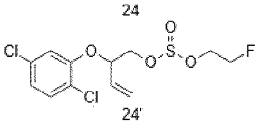
24'

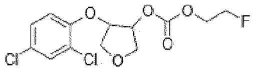
25
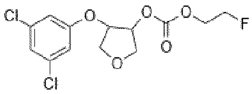
26
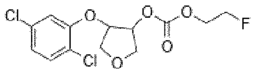
27
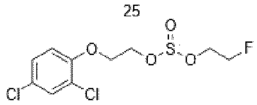
28
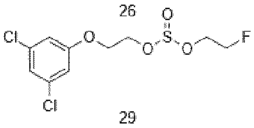
29
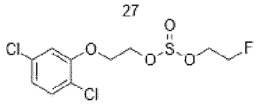
30
.

7. A method of controlling and/or killing pest eggs, or killing germs, wherein the compounds of any one of claims 1-6 are applied onto the pest eggs and/or germs.

8. The method according to claim 7, wherein when the compound is used for controlling and/or killing pest eggs of pests and/or killing germs, it is selected from one of the following compounds or a mixture of two or more thereof:

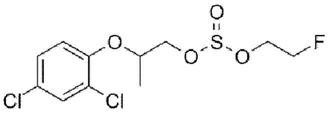
1
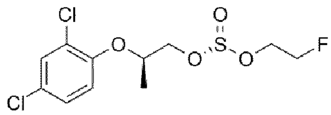
1-(R,R)
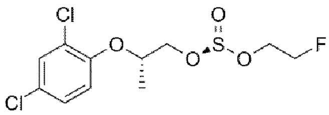
1-(S,S)

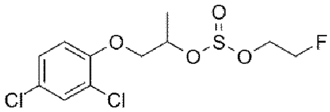
1'
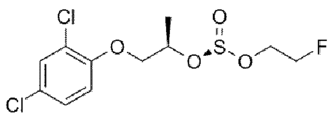
1'-(R,S)
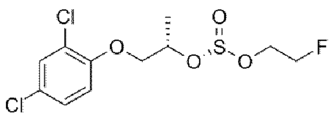
1'-(S,R)

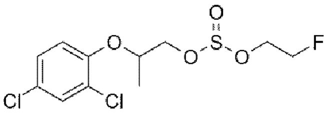
1
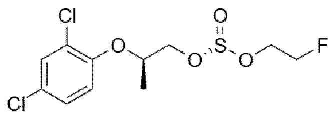
1-(R,R)
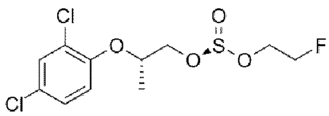
1-(S,S)

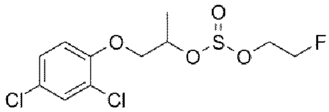

1'

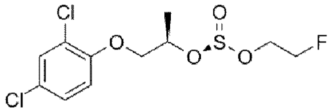

1'-(R,S)

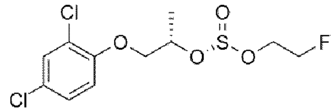

1'-(S,R)

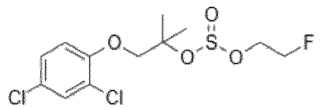

10

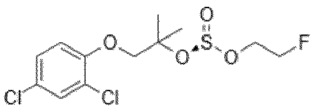

S-10

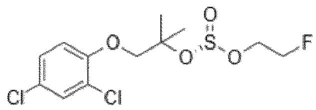

R-10

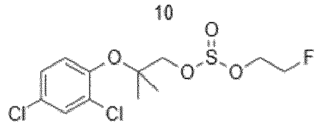

10'

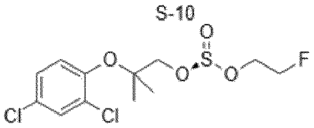

S-10'

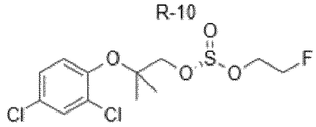

R-10'

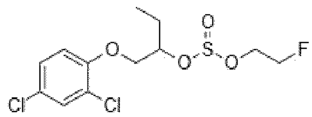

19

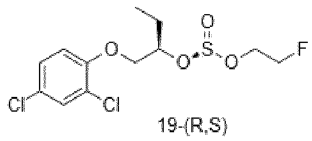

19-(R,S)

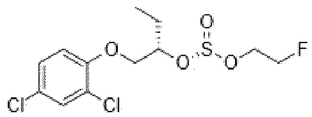

19-(S,R)

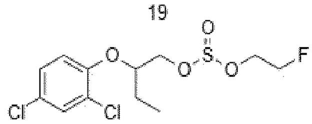

19'

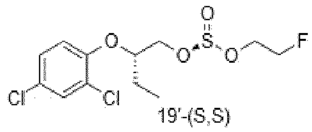

19'-(S,S)

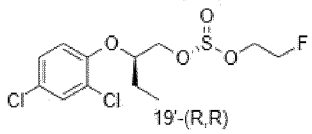

19'-(R,R)

**9.** The method according to claim 7, wherein the pest eggs are originated from insects of *Thysanoptera, Hemiptera, Lepidoptera, Coleoptera,* animals of *Tetranychidae, Tenuipalpidae, Eriophyidae, Tarsonemidae, Pyemotidae, Penthaleidae* or *Cheyetidae,* and the germs comprise fungi and bacteria.

**10.** The method according to claim 7, wherein the pest eggs are originated from *Frankliniella intonsa, Thrips tabaci Lindeman, Taeniothrips distalis Karn, Stenchaetothrips biformis, Thrips hawaiiensis Morgan, Thrips palmi Karny, Frankliniella occidentalis, Thrips japonicus Bagnall, Thrips serratus Kobus, Frankliniella tenuicornis Uzel, Scirtothrips dorsalis Hood, Heliothrips haemorrhoidalis Bouche, Scirtothrips dorsalis Hood, Scolothrips sexmaculatus Pergande, Cnaphalocrocis medinalis, Bemisia tabaci Gennadius, Trialeurodes vaporariorum, Aleurocanthus spiniferus, Dialeurodes citri Ashm, Bemisia myricae Kuwana, Aleurocybotus indicus, Aleurodicus dispersus, Oligonychus baipisongis, Oligonychus karamatus, Oligonychus rubicundus, Harmonia axyridis, Cerambycidae, Coccinellidae, Lampyridae, Scarabaeidae, Mylabris phalerata, Allomyrina dichotoma, Buprestidae, Melyridae, Scarabaeidae, Lucanidae, Elateridae, Dytiscidae, Sitophilus oryzae, Eotetranychus albus, Eotetranychus bailae, Eotetranychus camelliae, Tetranychus neocaledonicus, Tetranychus phaselus, Tetranychus urticae, Tetranychus cinnabarinus, Schizotetranychus baltazarae, Schizotetranychus bambusae, Schizotetranychus elongatus, Mixonychus aestiva, Mixonychus ganjuis, Panonychus citri, Panonychus caglei, Allonychus bambusae, Allonychus wuyinicus, Stigmaeopsis celarius, Mononychellus georgicus, Acanthonychus jiangfengensis* and *Amphitetranychus viennensis,* and the germ is Magnaporthe grisea.

**11.** The method according to claim 7, wherein the sulfite compound is applied in a concentration of no less than 0.1 ppm.

**12.** The method according to claim 7, wherein when in use, the sulfite compound is prepared into an agricultural product, which further comprises one or more accessories such as a dispersant, a wetting agent, a binder, an emulsifier, a stabilizer and a solvent.

**13.** The method according to claim 12, wherein the dosage form of the product is missible oil, suspension concentrate, wettable powder, powder, granule, water aqua, mother liquor or mother powder.

**14.** A pesticide composition, wherein the compound of formula (A) is used as the active substance.

**15.** The pesticide composition according to claim 14, wherein, it further comprises a mixture of ginger rhizome extract and

Kaempferia galanga L rhizome extract in a ratio of 7:3, wherein the ginger rhizome extract is obtained by extracting the ginger rhizome with ethanol:ethyl acetate=1-4:1; and the Kaempferia galanga L rhizome extract is a volatile oil from Kaempferia galanga L rhizome.

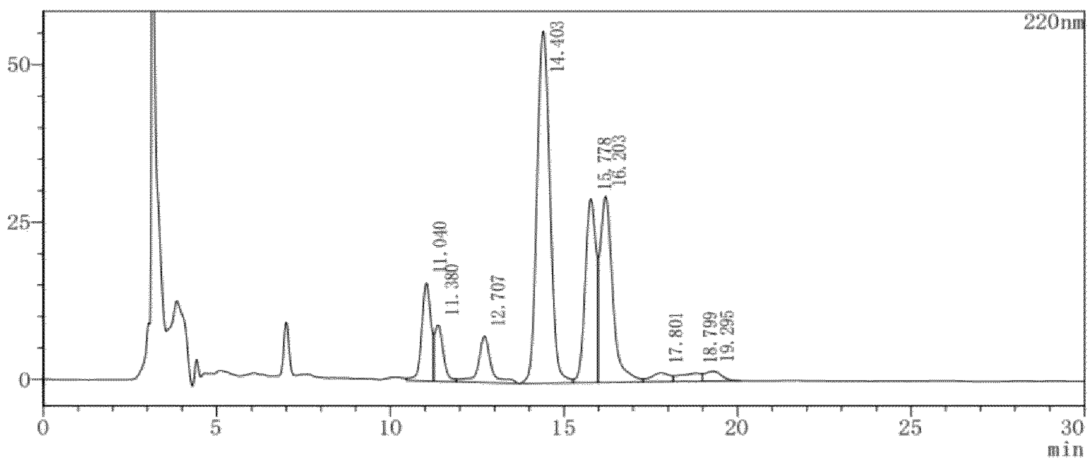


Figure 1

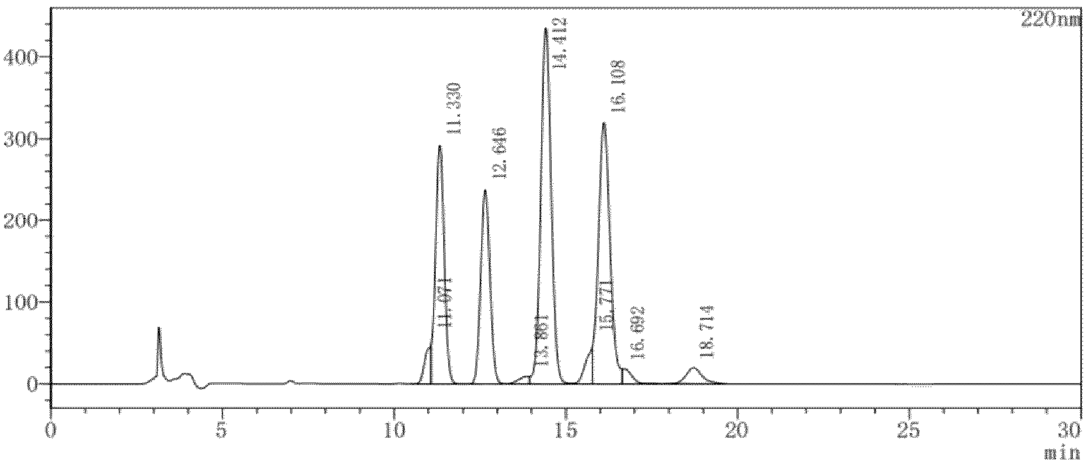


Figure 2

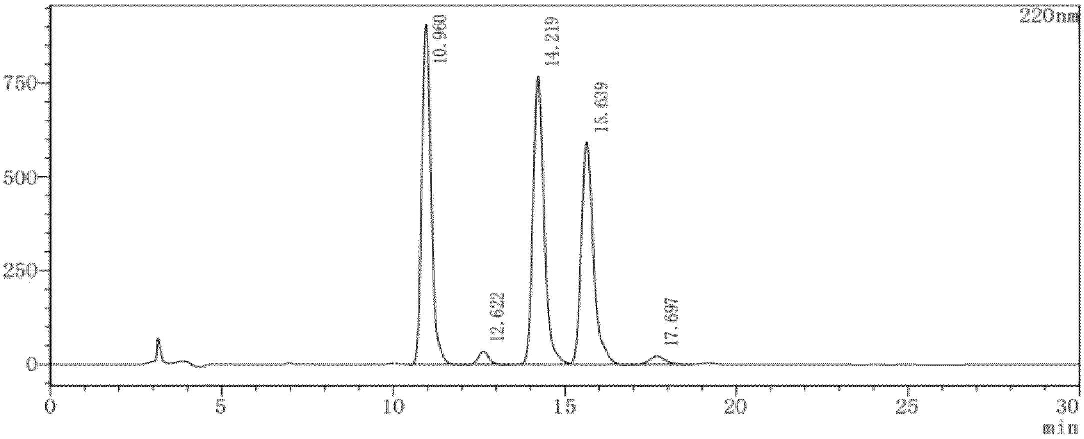


Figure 3

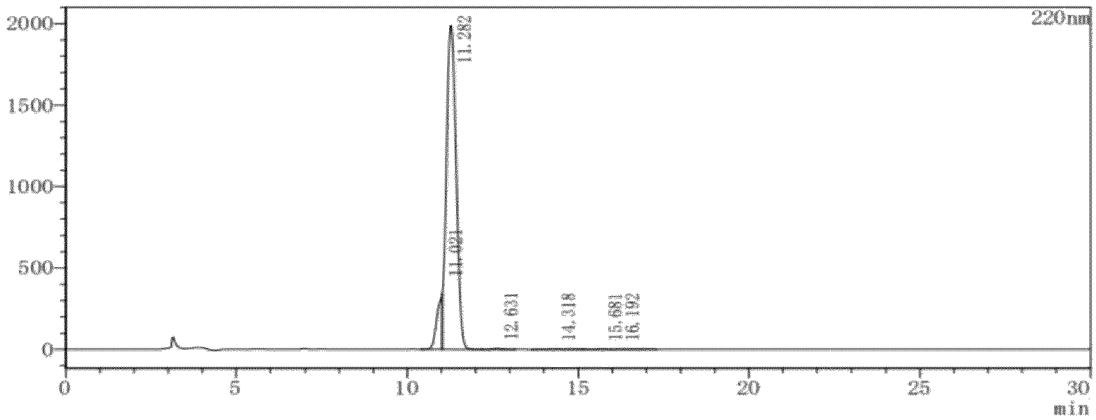


Figure 4

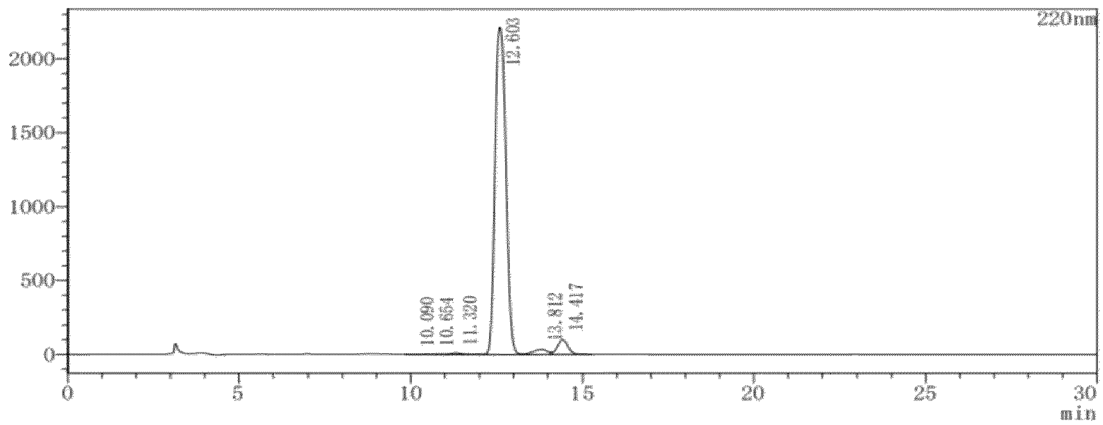


Figure 5

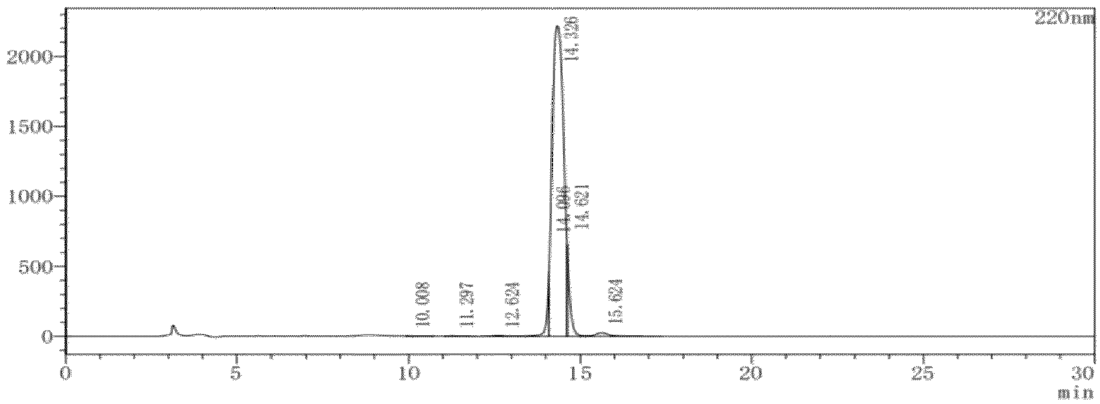


Figure 6

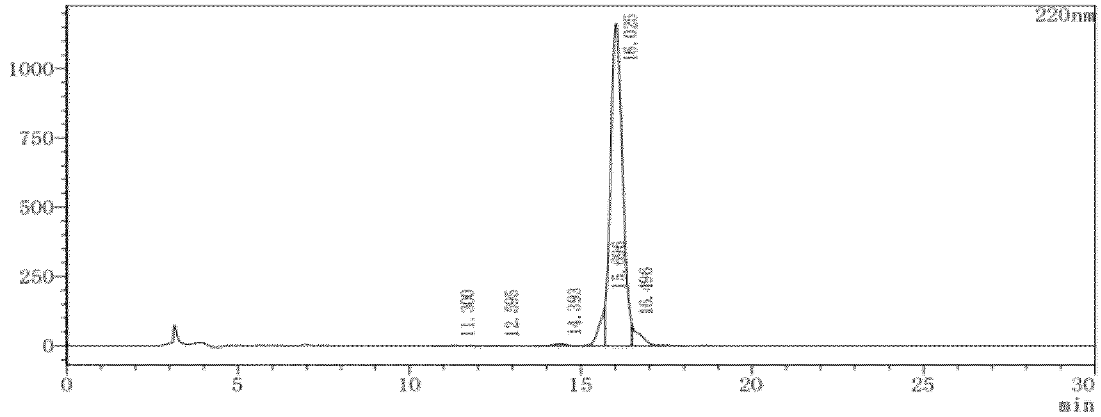


Figure 7

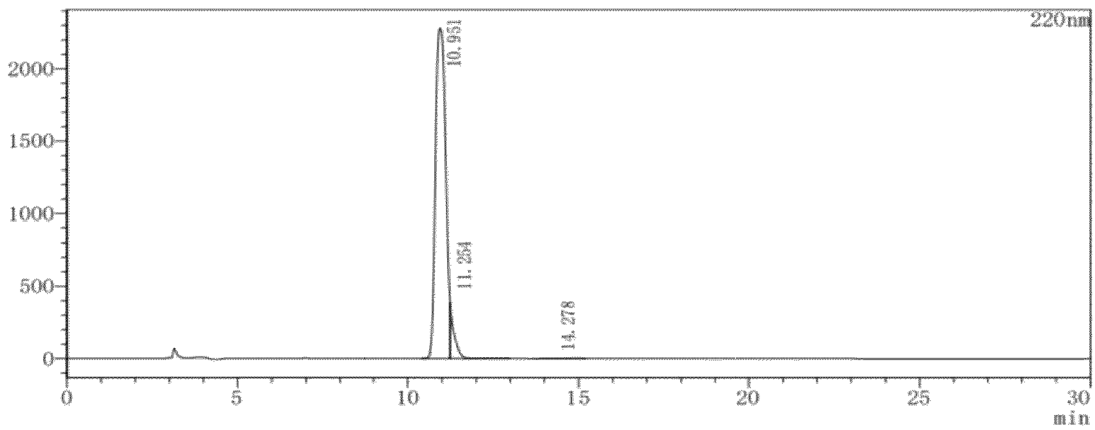


Figure 8

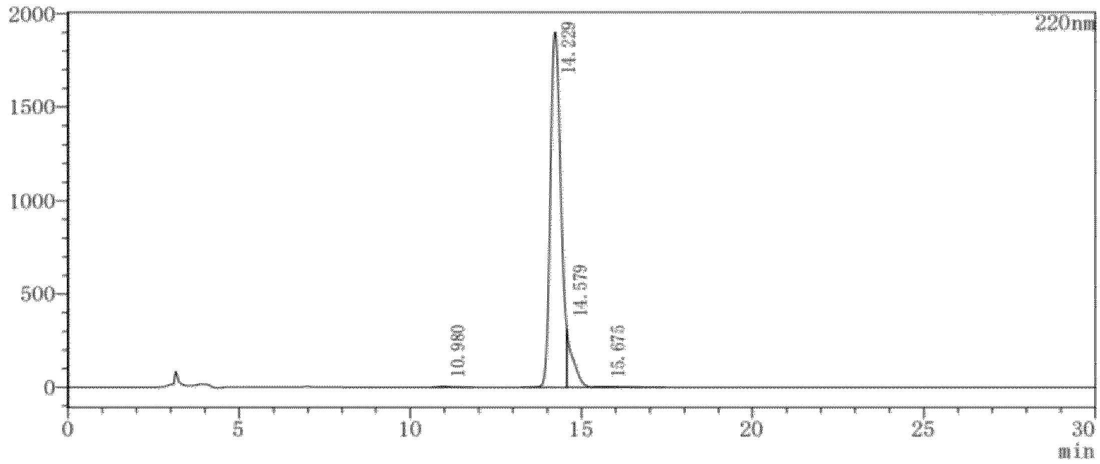


Figure 9

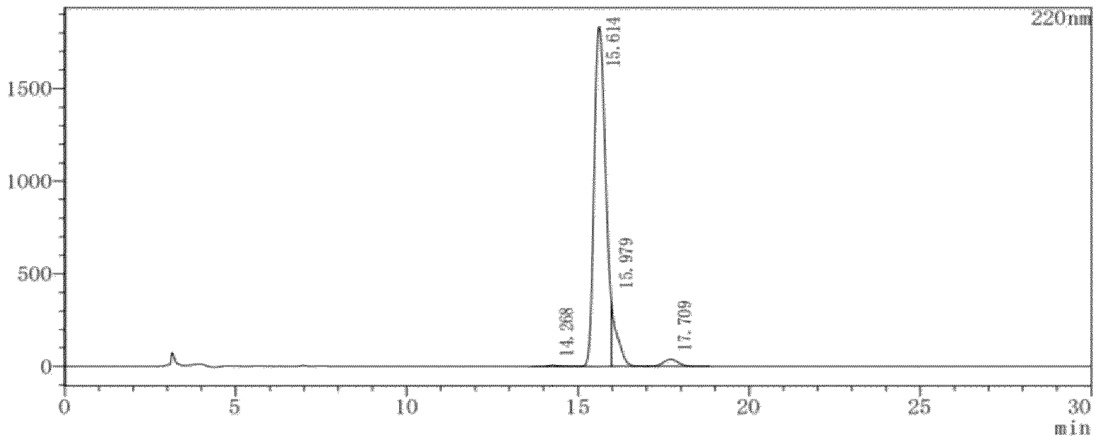


Figure 10

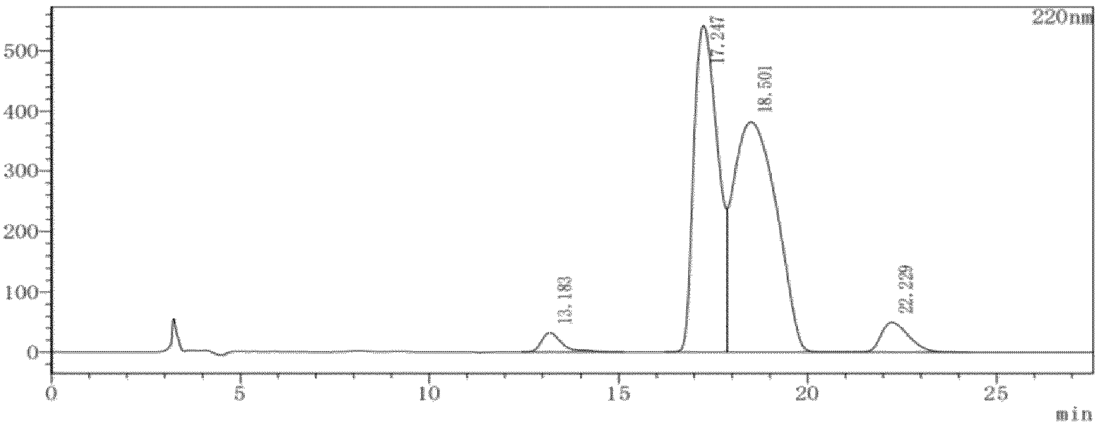


Figure 11

Europäisches Patentamt
European Patent Office
Office européen des brevets

# EUROPEAN SEARCH REPORT

Application Number
EP 25 16 9843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 118 666 717 A (UNIV NANKAI; SHANDONG UNITED PESTICIDE IND CO LTD) 20 September 2024 (2024-09-20) | 1-14 | INV. C07C301/00 C07D307/20 A01N41/02 A01N43/08 |
| A | * the whole document * | 15 | |
| X | CN 119 019 293 A (HENAN LANCHUANGZUOWU SCIENCE CO LTD) 26 November 2024 (2024-11-26) | 1-14 | |
| A | * the whole document * | 15 | |
| X | CN 118 373 760 A (SHANDONG DERUIKE CHEMICAL TECH CO LTD) 23 July 2024 (2024-07-23) | 1-6,14 | |
| A | * the whole document * | 15 | |
| X | CN 118 146 122 A (SHANDONG DERUIKE CHEMICAL TECH CO LTD) 7 June 2024 (2024-06-07) | 1-6,14 | |
| A | * the whole document * | 15 | |
| X | US 2 529 494 A (HARRIS WALTER D ET AL) 14 November 1950 (1950-11-14) * the whole document * | 1-6 | TECHNICAL FIELDS SEARCHED (IPC) |
| X | US 2 529 493 A (HARRIS WALTER D ET AL) 14 November 1950 (1950-11-14) * the whole document * | 1-6 | C07C C07D A01N |

-/--

The present search report has been drawn up for all claims

2

| Place of search | Date of completion of the search | Examiner |
|---|---|---|
| The Hague | 30 September 2025 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 25 16 9843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MERLIN I. ET AL: "Plant extracts for the management of two spider mite, Tetranychus urticae Koch on jasmine (Jasminum sambac)", PEST MANAGEMENT IN HORTICULTURAL ECOSYSTEMS, vol. 29, no. 1, 1 January 2023 (2023-01-01), pages 172-176, XP093310274, ISSN: 0971-6831, DOI: 10.5958/0974-4541.2023.00027.9 * the whole document * ----- | | |
| T | MOAZENI MOHAMMAD ET AL: "Ovicidal effect of the methanolic extract of ginger (Zingiber officinale)onFasciola hepaticaeggs: an in vitro study", JOURNAL OF PARASITIC DISEASES, SPRINGER INDIA, INDIA, vol. 40, no. 3, 9 September 2014 (2014-09-09), pages 662-666, XP036656642, ISSN: 0971-7196, DOI: 10.1007/S12639-014-0554-Z [retrieved on 2014-09-09] * the whole document * ----- | | TECHNICAL FIELDS SEARCHED (IPC) |

-/--

The present search report has been drawn up for all claims

2

| Place of search | Date of completion of the search | Examiner |
|---|---|---|
| The Hague | 30 September 2025 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 25 16 9843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | BIÑAS ENRIQUE E: "Efficacy of rhizome crude extracts organic pesticide against insectpests and its impact on glutinous corn (Zea mays L. var. ceratina) Production", JOURNAL OF BIODIVERSITY AND ENVIRONMENTAL SCIENCES (JBES), 30 April 2021 (2021-04-30), XP093310281, ISSN: 2220-6663 Retrieved from the Internet: URL:https://www.innspub.net/wp-content/uploads/2022/05/JBES-V18-No4-p27-36.pdf> * the whole document * ----- | | |

TECHNICAL FIELDS SEARCHED (IPC)

The present search report has been drawn up for all claims

2

| Place of search | Date of completion of the search | Examiner |
|---|---|---|
| The Hague | 30 September 2025 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 25 16 9843

This annex lists the patent family members relating to the patent documents cited in the above-mentioned European search report. The members are as contained in the European Patent Office EDP file on
The European Patent Office is in no way liable for these particulars which are merely given for the purpose of information.

30-09-2025

| Patent document cited in search report | Publication date | Patent family member(s) | Publication date |
|---|---|---|---|
| CN 118666717 A | 20-09-2024 | NONE | |
| CN 119019293 A | 26-11-2024 | NONE | |
| CN 118373760 A | 23-07-2024 | NONE | |
| CN 118146122 A | 07-06-2024 | NONE | |
| US 2529494 A | 14-11-1950 | NONE | |
| US 2529493 A | 14-11-1950 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Absolute configuration of glycosyl sulfoxides. *Tetrahedron: Asymmetry*, 2010, vol. 21 (15), 1830-1832
- Absolute configuration of glycosyl sulfoxides. *Tetrahedron: Asymmetry*, 2010, vol. 21 (15), 180-1832, https://doi.org/10.1016/j.tetasy.2010.06.019